# EUROPEAN PATENT APPLICATION

(11) **EP 2 417 989 A1**
(43) Date of publication of application: **15.02.2012**
(21) Application number: 10753435.6
(22) Date of filing: 03.03.2010
(51) Int. Cl.: A61L 27/00

(54) **MEDICAL MATERIAL FOR IN VIVO IMPLANTATION CONTAINING SOFTENING AGENT AND/OR MOISTURIZING AGENT , METHOD FOR CONTROLLING CONTENT OF SOFTENING AGENT AND/OR MOISTURIZING AGENT IN THE MEDICAL MATERIAL, AND METHOD FOR PRODUCING THE MEDICAL MATERIAL FOR IN VIVO IMPLANTATION**

(30) Priority: 18.03.2009 JP 2009066278
(71) Applicant: NICEM LTD, Yokohama 236-0005 (JP)
(72) Inventor: NOISHIKI Yasuharu, Yokohama-shi Kanagawa 236-0005 (JP)
(74) Representative: McCluskie, Gail Wilson
(86) International application number: PCT/JP2010/053936
(87) International publication number: WO 2010/106943

(57) **Abstract**

Provided are a medical material for in vivo implantation which causes no retention of an exudates in the surroundings and yet shows a flexibility; a method for controlling the content of a softening agent and/or a moisturizing agent in the medical material; and a method for producing the medical material for in vivo implantation. When a hybrid medical material for in vivo implantation, which comprises a bioabsorbable material containing a softening agent and/or a moisturizing agent and a non-bioabsorbable porous base material, contains large amount of the softening agent and/or a moisturizing agent, retention of an exudates occurs in the surroundings of the medical material. It is found out that this phenomenon is a side effect of the softening agent and/or a moisturizing agent. To prevent this side effect, a medical material for in vivo implantation in which the content of a softening agent and/or a moisturizing agent is controlled less than 20 wt%; a method for controlling said content; and a method for producing a medical material for in vivo implantation are provided.

## Description

### FIELD OF THE INVENTION

The present invention relates to a hybrid medical material for in vivo implantation, which is used for surgical operation of blood vessel, trachea, blood vessel wall, heart wall, pericardium, chest wall or peritoneum, comprising a bioabsorbable material and a non-bioabsorbable porous substrate. Especially, in a case when large amount of a softening agent and/or a moisturizing agent (can be shortened simply as softening agent) are contained in said medical material for in vivo implantation, a phenomenon of retention of exudates to a circumference of the medical material causes. In the present invention, the inventor of the present invention indicate that the reason of the phenomenon is a side effect of the softening agent and/or moisturizing agent, and for the purpose to suppress the side effect, the inventor accomplish the present invention. That is, the present invention relates to a medical material for in vivo implantation whose content of the softening agent and/or moisturizing agent is minimized as small as possible, a method to adjust the content of the softening agent and/or moisturizing agent in said medical material for in vivo implantation and a method for preparation of the medical material for in vivo implantation.

### DESCRIPTION OF PRIOR ARTS

As a medical material which is used at surgical operation of blood vessel, trachea, blood vessel wall, heart wall, pericardium, pleural or peritoneum, a hybrid medical material for in vivo implantation comprising combination of a non-bioabsorbable porous substrate such as cloth or tube and a bioabsorbable material can be mentioned. For example, in a case of artificial blood vessel or a patch material for heart wall, there is so called a coated artificial blood vessel or a coated patch characterized that a bioabsorbable material is coated on a porous structural substrate made of polyester fibers, which is non-bioabsorbable porous material, intending to protect leak of blood or body fluid from interstices of fibers, and these are a hybrid medical material for in vivo implantation in which a bioabsorbable material is used as a coating material. In prior arts, bio originated substance such as collagen or gelatin provides good sites to a cell, and because cell affinity of it is high, it is applied as a bioabsorbable material.

As the prior arts relating artificial blood vessel which uses collagen or gelatin as coating materials for bioabsorbable material, Patent Documents 1-9 are published.

As shown in Patent Documents 10-19, in a case of bovine originated collagen, since there is an apprehension of BSE (so called Bovine Spongiform Encephalopathy), hydrophobic synthetic polymer materials such as poly-L-latic acid, poly-D,L-latic acid, poly ε -caprolactone, polyglycolic acid, polymerized products thereof or mixture thereof are paid attention as substitutes of collagen or gelatin. In documents mentioned below (Patent Documents 10-19), hydrophobic synthetic polymer materials are applied as a coating material.

In cases of these hydrophobic materials, it is not necessary to consider dry state, however, since in a case of porous medical material for in vivo implantation on which hydrophilic material such as collagen is coated, said hydrophilic material is hardened in dry condition and handling deteriorates and becomes easy to crack, flexibility is provided by containing a softening agent or a moisturizing agent. In above mentioned Patent Document 1-7, glycerin, sorbitol or mannitol acts as a softening agent or a moisturizing agent and there is a recitation referring uses of these compounds. Since these softening agents or moisturizing agents are not cytotoxic for a living body, these compounds are not problem and conventionally used. In almost all clinically use products of today, glycerin which has both future of softening agent and moisturizing agent is contained.

In the first place, current situation of a medical material for in vivo implantation containing glycerin which is a typical softening agent and/or moisturizing agent is explained.

Although glycerin is liquid, does not vaporize naturally. Therefore, since product which contains glycerin can maintain flexibility even if the product is preserved in dry condition, further glycerin does not have cytotoxity, in all medical materials for in vivo implantation being on the market, which enmeshes bioabsorbable material to non-bioabsorbable substrate, glycerin is contained, and the medical materials for in vivo implantation are packed and sterilized in clinically usable state. Considering said situation, in conventional arts, it is conjectured that a problem regarding amount of glycerin content is not raised. If washing is necessary to decrease the content of glycerin, illustration that the product must be washed before clinical use must be mentioned in explanatory note or brochure. Or the product must be washed before sterilization and packed, for the purpose not to trouble a clinical doctor. However, actually, there is no illustration about washing in explanatory note, and a medical doctor picks out the medical material for in vivo implantation from a sterilized package and clinically uses it as it is. That is, a medical material for in vivo implantation comprising non-bioabsorbable porous material substrate and bioabsorbable material prepared by conventional art is packed and sterilized on the assumption that to be used by containing glycerin. Accordingly, the product is clinically used by containing glycerin and is implanted in a living body. According to the fact, side effect of glycerin, especially, side effect caused by containing large amount of glycerin is not considered as a problem.

However, glycerin has a property of strong water absorbance beside a property of maintaining flexibility, and the inventor of the present invention has found out that the property of strong water absorbance causes a side effect. This fact is specifically explained as below. When a medical material contains large amount of glycerin, glycerin contained in the medical material absorbs water in body fluid after the medical material is implanted in a living body and causes high water absorbing state, and the state has a possibility to act as a cytotoxic side effect. Concretely, since glycerin has a property to draw water in succession from surroundings, in a case when large amount of glycerin is contained, there is a possibility to draw excessive water, and glycerin is eluted in the drawn water, then eluted glycerin further draws water from surroundings and forms state of retention of exudates. If eluted glycerin is introduced into a tissue surrounding the medical material, water is drawn in at the position and forms interstitial edema. In such a condition, fibroblasts, which acts strongly to heal tissue, looses tissue healing force in a circumference of the medical material and tissue healing delays, because fibroblasts have a property that "activity stops at high water absorbing condition". Further, the tissue becomes defenseless state to bacterium infection, and forms dangerous state to cause infection. The inventor of the present invention found out above mentioned facts and proposed it as an object to be dissolved in the present invention.

In a medical material for in vivo implantation prepared by conventional art, glycerin is contained as a typical softening agent, and it is important to know the content of glycerin accurately. For the purpose to measure glycerin content accurately, "48 hours washing method" mentioned below is used as a method to extract glycerin completely.

As an example, an artificial blood vessel coated by collagen or gelatin is mentioned. The adequate length, specifically 10cm length of the artificial blood vessel is cut out and weight (a) in dry state is measured. To obtain dry state of the artificial blood vessel, it is convenience to use a lyophilizer. This weight is total weight of non-bioabsorbable porous substrate, hydrophilic bioabsorbable material and glycerin. Since bioabsorbable material is crosslinked, weight of crosslinking agent is included in (a). Then the specimen of the artificial blood vessel is dipped in 1 liter of distilled water and permeated for 48 hours. Distilled water is exchanged to new one every 8 hours. At the exchange process of distilled water, the specimen of the artificial blood vessel is wrapped in gauze, and water contained in the specimen is removed by pushing it lightly. By dipping it in distilled water again, water in the artificial blood vessel wall can be exchanged. By these operations glycerin can be eluted completely from the artificial blood vessel to distilled water after 48 hours. After that, the specimen of the artificial blood vessel is dried up completely and the weight of it is measured (b). Since glycerin is removed, the artificial blood vessel looses flexibility and hardened. Difference between (a) which is the weight before washing, and (b) is the weight of glycerin (c). By these values, weight % of (c) contained in (a) can be calculated.

Regarding weight of softening agent such as glycerin to be used in prior arts, there is a recitation in Patent Document 1-3 that 4-12% of glycerin is contained in solution containing 0.5-5% of collagen, therefore, content of glycerin is corresponding to 8-24 times to amount of collagen. In Patent Document 4, amount of glycerin is not mentioned. In Patent Documents 5 and 6, use of glycerin in range of 8-30% in solution containing 1-3% of collagen is mentioned. That is, indicates use of 8-24 times of glycerin to collagen. In Patent Document 7, there is a recitation reciting that 0,3-0.5g of glycerin is used to 1g of artificial blood vessel cloth. That is, amount of glycerin is corresponding to 23-33% of total weight. In any case, since content of glycerin is large, risk by side effect caused by use of large amount of glycerin is not considered in conventional art.

Products being on the market, which is based on prior arts, are mentioned as examples. In a case of Gelweave artificial blood vessel, which is a product of Vascutek Co., Ltd. (Scotland) and a typical gelatin coated artificial blood vessel, weight % of glycerin content to the total weight of artificial blood vessel is about 29%. In a case of Hemashield artificial blood vessel, which is a product of Boston Scientific Co., Ltd. (U.S.A. New Jersey State) and a typical collagen coated artificial blood vessel being on the market, weight % of glycerin content to the total weight is about 41.8% and that of Interguard artificial blood vessel, which is a product of Inter Vascular Co., Ltd. (U.S.A. Florida State), is about 22.1%. That is, the content of glycerin is not decided by strict inspection and consideration. Further, in prior arts, there is no careful consideration to decrease the containing amount of glycerin as small as possible, and products by prior art based on said consideration cannot be found out.

In the meanwhile, in actual clinic, a problem that retention of exudates causes in a circumference of coated artificial blood vessel after it is implanted in a living body was pointed out. As reported in Chest Surgical 1993;46:1093-98 (Not Patent Document 20), it is pointed out that the ground of said problem can be antigen-antibody reaction or can be containing of endotoxin, however, true ground of the problem is not made clear yet. When exudates retains in a circumference of artificial blood vessel, cell migration to artificial blood vessel wall is protected and formation of neointima delays and the artificial blood vessel becomes to be easily contaminated. Further, when the coating substance is decomposed in the state that cell migration to artificial blood vessel wall is protected, there is a dread of blood leaking and have a possibility to cause unexpected complication. The inventor of this invention has found out above mentioned facts, investigated the ground of retention of exudates, and considered that the protection of such facts is necessary. However, although a product which minimizes retention of exudates is strongly desired in actual clinic, products based on conventional art were still continuously used up to the present time, because the ground of the problem was not clear.

The inventor of the present invention, pay attention that polyhydric alcohol specifically glycerin used in prior arts, which is used aiming to provide flexibility to dried collagen, has strong water absorbing ability, and the inventor becomes to be anxious about following side effect. That is, when glycerin is implanted in a living body and draw excessive water from surrounding tissue, a problem of retention of exudates which cannot be absorbed in surrounding tissue will be caused in a circumstance of medical material for in vivo implantation, and the inventor investigates about side effects that high water absorbency of glycerin has. Thus, the inventor started the study to dissolve the problem.

In the present invention, amount of glycerin contained in materials is decreased gradually, and finely investigate a material which does not contain glycerin. As the results, the inventor confirmed following facts. That is, amount of retention of exudates can be decreased by dropping concentration of glycerin, retention of exudates is not caused under certain amount of glycerin, and retention of exudates is not caused at all when glycerin is not contained. Accordingly, the inventor noticed that the problem will be dissolved by controlling amount of glycerin which was not recognized as a ground of problem in the conventional arts.

Concurrently, same investigation was made on sorbitol or mannitol, which is a kind of polyhydric alcohol having softening property and moisturizing property like as glycerin, and, it became clear that these compounds have strong water absorbing ability and draw large amount of water from surrounding tissue and cause side effect same as glycerin. Accordingly the inventor reported that it is necessary to restrict the amount of use of these softening agents and/or moisturizing agents.

However, since softening agent and/or moisturizing agent is necessary to maintain flexibility of medical materials which have a possibility to be preserved in dry condition, certain amount of these compounds is used. If the amount of these compounds to be contained in medical materials is decreased, medical materials become to lack flexibility and to be hardened, and handling property deteriorates. Therefore, in actual clinic, these medical materials become to be hard for actual use. When these medical materials are used in said hardened state, sometimes cause a problem of destruction. Therefore, if amount of glycerin is aiming to decrease, it is necessary to take countermeasure for the purpose to provide flexibility with the medical materials.

For the purpose to decrease amount of softening agent, following subjects must be dissolved. That is, what is minimum necessary amount of additives such as softening agent, how to provide flexibility to medical materials in actual clinic in a case when amount of additives such as softening agent is decreased, what kind of other special property must be provided to a non-bioabsorbable substrate, and in what condition a bioabsorbable material must be combined with a non-bioabsorbable substrate.

In the present invention, the inventor continued eagerness study to decrease total amount of a softening agent contained in a medical material for in vivo implantation and to dissolve a problem caused by decrease of softening agent, and found out a point for improvement.

Further, the inventor found out following facts. That is, even if a medical material for in vivo implantation containing excessive amount of glycerin, it is possible to drop concentration of glycerin by washing with a solvent of glycerin, concretely electrolyte solution such as physiological saline, isotonic solution or distilled water at preparation process or at actual use in actual clinic, if concentration of glycerin is dropped, retention of exuded liquid does not cause. While, the inventor made clear that, even if glycerin is washed out, that flexibility of it can be maintained by solvent. That is, solvent maintain water containing condition of the medical material for in vivo implantation and maintains flexibility of it, and handling of it does not deteriorate at actual use in actual clinic. In the present invention, the inventor found out that when a medical material for in vivo implantation is actually used in actual clinic in the state containing large amount of glycerin, even if flexibility by glycerin is necessary to keep good handling ability, a problem of retention of exudates causes, and the problem can be dissolved by making the amount of glycerin under certain amount by washing with solvent.

### PRIOR ARTS

### Patent Documents

Patent Document 1: U.S.P 4,842,575 publication
Patent Document 2: U.S.P 5,108,424 publication
Patent Document 3: U.S.P 5,197,977 publication
Patent Document 4: U.S.P 5,851,229 publication
Patent Document 5: U.S.P 6,177,609 publication
Patent Document 6: U.S.P 6,299,639 publication
Patent Document 7: U.S.P 5,584,875 publication
Patent Document 8: U.S.P 6,368,347 publication
Patent Document 9: U.S.P 6,670,096 publication
Patent Document 10: U.S.P 4,990,158 publication
Patent Document 11: JP 2004-313310 publication

### Non Patent Documents

Non Patent Document 1: ASAIO Trans. 1988 Jul-Sep;34:789-93
Non Patent Document 2: J Surg Res. 2001 Feb;95:152-60
Non Patent Document 3: J Biomater. 1996 Apr; 10:309-29
Non Patent Document 4: ASAIO Trans. 1988 Jul-Sep;34:789-93
Non Patent Document 5: J Surg Res. 2001 Feb;95:152-60
Non Patent Document 6: J Biomater. Sci Polym Ed.2003;14:1057-75
Non Patent Document 7: J Artif Organs 2002 Aug;25:777-82
Non Patent Document 8: J Artif Organs 1999 Dec;22:843-53

### BRIEF SUMMARY OF THE INVENTION

### OBJECT OF THE INVENTION

The object of the present invention is to suppress side effect of softening agent and/or moisturizing agent of polyhydric alcohol such as glycerin, sorbitol or mannitol and how to obtain flexibility when amount of softening agent and/or moisturizing agent is decrease. The present invention is to provide a medical material for in vivo implantation suppressing amount of softening agent and/or moisturizing agent, having flexibility and not causing retention of exudates to circumference of the medical material, a method to control the amount of content of softening agent and/or moisturizing agent and a method for preparation of a medical material for in vivo implantation which perform flexibility by containing water.

### BRIEF ILLUSTRATION OF THE INVENTION

The inventor of the present invention has found out that above mentioned object can be accomplished by controlling optimized amount of softening agent and/or moisturizing agent to less than 20 weight %, and the essential point of this invention is an idea to dissolve said object.

The first measure to accomplish above mentioned object is to provide a medical material whose content of softening agents is less than 20 weight % in a medical material for in vivo implantation comprising a bioabsorbable material and a non-bioabsorbable porous substrate. And condition of said "less than 20 weight %" is in actual clinic use.

The second measure to accomplish above mentioned object is to provide a medical material which is possible to decrease the amount of said softening agent to less than 20 weight % by washing with physiological saline before actual use in clinic, in a medical material for in vivo implantation comprising a bioabsorbable material and a non-bioabsorbable porous substrate.

The third measure to accomplish above mentioned object is to investigate a method to provide with flexibility to a medical material for in vivo implantation by containing water, because flexibility which is necessary to a medical material deteriorates when the amount of said softening agent is adjusted to less than 20 weight %. Concretely, the measure is to make a bioabsorbable material possess electric charge group, or to provide with a bioabsorbable material which meets to a condition whose hydroxyl value is more than 50KOH mg/g.

The fourth measure to accomplish above mentioned object is an investigation regarding crosslinking method of a bioabsorbable material. Concretely, the investigation is to change a crosslinking method from conventional crosslinking methods i.e. crosslinking by glutaraldehyde or formaldehyde to a method by poly-epoxy crosslinking.

The fifth measure to accomplish above mentioned object is to provide with a non-bioabsorbable substrate having flexibility by itself, because flexibility necessary to medical materials deteriorates when amount of softening agent is suppressed to minimum value. When fibrous materials are used in conventional arts, if density of fiber is elevated, substrate becomes hard. In the present invention, a method to provide a substrate with flexibility by not performing closest packing condition but by filling very fine fibers between interstices of fibers so as to draw out slipping property of the very fine fibers.

The sixth measure to accomplish above mentioned object is to provide with a method for preparation of a medical material for in vivo implantation comprising a bioabsorbable material and a non-bioabsorbable porous substrate containing a softening agent and/or a moisturizing agent comprising, adjusting content of the softening agent and/or the moisturizing agent to less than 20 wt% by washing the medical material for in vivo implantation with electrolyte solution such as physiological saline, isotonic solution or distilled water, or by impregnating electrolyte solution such as physiological saline, isotonic solution or distilled water into the medical material for in vivo implantation.

The seventh measure to accomplish above mentioned object is performed by considering that at the impregnating and enmeshing process of a bioabsorbable material into porous structure of a non-bioabsorbable porous substrate, if a softening agent and/or a moisturizing agent is contained in the bioabsorbable material, even if the amount is small, the bioabsorbable material swells by water absorption and becomes difficult to enter into structure of the non-bioabsorbable porous substrate. That is, the seventh measure to accomplish above mentioned object is to provide with a method for preparation of a medical material for in vivo implantation comprising a bioabsorbable material and a non-bioabsorbable porous substrate containing a softening agent and/or a moisturizing agent comprising, suppressing excessive swelling of the bioabsorbable material by dissolving it at isoelectric point or nearby the point, or containing salt and/or alcohol in the dissolved solution or suspension, and injecting the solution or suspension into porous structure so as large amount of the bioabsorbable material, whose excessive swelling is suppressed, to enmesh with the non-bioabsorbable porous substrate.

### EFFECT OF THE INVENTION

In the present invention, paragraph of "comprising a bioabsorbable material and a non-bioabsorbable porous substrate" means hybrid medical material for in vivo implantation, prepared by coating a bioabsorbable material on a non-bioabsorbable porous substrate, by pushing a bioabsorbable material into a non-bioabsorbable porous substrate or by enmeshing a bioabsorbable material with a non-bioabsorbable porous substrate. In a medical material for in vivo implantation comprising a bioabsorbable material and a non-bioabsorbable porous substrate of the present invention, by suppressing content of a softening and/or a moisturizing agent less than 20 wt%, retention of exudates caused in surrounding of said medical material can be controlled and cytotoxic effect or complication caused by retention of exudates can be minimized. And as a conception to make up an inconvenience caused by shortage of amount of a softening and/or a moisturizing agent, a method to maintain flexibility by washing the medical material for in vivo implantation with solvent such as electrolyte solution i.e. physiological saline, isotonic solution or distilled water or impregnating electrolyte solution such as physiological saline, isotonic solution or distilled water into the medical material for in vivo implantation can be used.

By performing said conception, the medical material for in vivo implantation of the present invention can be used as a substitute or a part of a tubular viscus or a tubular tissue in vivo such as an artificial blood vessel or an artificial trachea, or can be used in patch form on organs or tissues in vivo as an artificial pericardium, an artificial heart wall, an artificial abdominal wall, an artificial blood vessel wall, an artificial trachea wall, an artificial pleura, an artificial dura matter or an artificial urinary bladder wall.

### PREFERRED ENBODIMENT OF THE INVENTION

The preferred embodiment of the present invention will be illustrated as follows by dividing into 7 essential points.

The essential point of the first invention of the present invention is to restrict content of a softening and/or a moisturizing agent to less than 20 wt% in medical material for in vivo implantation comprising a bioabsorbable material and a non-bioabsorbable substrate. In the prior arts, following softening agents and/or moisturizing agents were used.

That is, hydrophobic hydro carbon compound such as squaran or squaren, hydrophilic polyhydric alcohol such as glycerin, xylite (xylitol), di-glycerin, dipropyleneglycol, sorbitol (sorbit), mannitol (mannit), multitol, lactitol, opparatinit, erythritol, DL-sodiumpyrrolidonecarbonate, 1,3-butyleneglycol, propyleneglycol, polyethyleneglycol (carbowax), polyglycerin, U-jerry or derivatives thereof can be used. Further, trehalose, NMF (natural moisturizing factor), aqualizer-EJ, produe, mixed isomerized sugar (bentabaiden), amino acid, L-asparagic acid, L-sodium asparatate, DL-alanine, L-arginine, L-isoleucine, lysinehydrochloride (L-lysine hydrochloric acid), glycine (amino acetate), L-glutamine, L-glutamic acid, L-sodium glutamate, γ -aminobutyrate (piperidine), L-threonine, sericin, serine, L-tyrosine (L-tyrodine), L-tryptophan, L-valine, L-histidine hydrochloric acid salt , L-hydroxy proline (L-oxyproline), phenylalanine, L-proline, L-leucine, DL-pyrrolidone carboxylic acid (PCA), DL-sodium pyrrolidone carboxylate, lactic acid, sodiumlactate, urea, uric acid, acidic muco polysaccharides, extracted liquid from umbilical cord, extracted liquid from crista galli, hyaluronic acid, sodium hyaluronate, chondroitin sulfate natrium, glucuronic acid, glucurone, elastin, water-soluble elastin, intercellular lipid, sphingolipid (ceramide), HS-oil, keratin, hydrolysis keratin, keratin amino acid, cystine, L-methionine, cystine, sulfuric acid, deoxyribonucleic acid (DNA), ribonucleic acid (RNA), guanosine, guanine, phosphoric acid, ATP (adenosine triphosphate,' tryptophan adenosine, phosphoric acid, sodium riboflavate, phospholipid, lecithin, soybean phospholipid (soybean lecithin), soybean lysopholipid (lysolecithin), yolk yellow lecithin (yolk yellow phospholipid), enzyme, vegetable complex enzyme, albumin analysis enzyme, fat analysis enzyme (lipase) can be used.

The inventor of the present invention endeavored to decrease the amount of use of these compounds. In a case that the use of a softening agent and/or a moisturizing agent is necessary, the inventor tried to minimize the amount of it, and not the amount of it to exceed 20 wt%. According to the result by animal experiments, it is succeeded that the retention of exudates to circumference of the medical material can be suppressed when the amount of a softening agent and/or a moisturizing agent is restricted in a limit of less than 20 wt%. The limitation of less than 20 wt% means that the softening agent and/or the moisturizing agent is in said condition at the actual use in clinic. Consequently, it is sufficient that the content of the softening agent and/or the moisturizing agent is restricted to less than 20 wt% at the state that the medical materials is prepared, packed and sterilized so as to cope with the actual use in clinic.

The standard of "containing less than 20 wt%" is drawn from results of animal experiments. However, in any kind of investigation, it is pointed out as a problem that when results of animal experiments are extrapolated to human's clinical at any clinical study. In a case of animal experiment, it is general to use an animal in healthy state, however, in actual clinical case, since an aged patient whose metabolic ability is weakened is an object, these cases cannot be managed equally. Accordingly, also in the present invention, considering basic theory and phenomenon of retention of exudates, "amount of softening agent and/or moisturizing agent is less than 20 weight %" must be reconsidered.

In medical materials for in vivo implantation, comprising a bioabsorbable material containing softening agent and/or moisturizing agent such as glycerin and a non-bioabsorbable porous substrate, glycerin absorbs water from surroundings irrespective of amount of content of glycerin after implanted in living body. And the absorbed water dilutes glycerin in the medical materials, however, absorbable amount of water in the medical materials is limited, glycerin is diluted and mixed with water and exudes from the medical materials. The glycerin flows into tissue with water and mixed with electrolyte of surrounding tissue and metabolized as interstitial fluid. At this time, when nutritional state of a patient is good and concentration of protein such as albumin in blood is high, interstitial fluid is absorbed into capillary blood vessel with assistance of osmotic action, and transported by blood far from the medical materials. Thus the interstitial fluid surrounding the medical materials is transported gradually. And if renal function of the patient is also good, the interstitial fluid containing glycerin is excreted from a renal through a filter. That is, when nutritional state of a patient is good, metabolism activity is high and renal function is normal, water flow in tissue, namely flow of interstitial fluid is active, proper amount of interstitial fluid can be treated. However, when the amount is over the limit of treatment, interstitial fluid not transferred remains in circumference of the medical materials, flooded state of tissue, namely edema state is caused. In a case of a patient whose nutritional state is bad or renal function is bad, water always retains in tissue and is in state of swelling. Therefore, when medical materials containing glycerin is implanted in said patient, edema state is easily caused and interstitial fluid retains in circumference.

Since animals used for experiment is healthy and metabolism activity is high, as indicated in experimental result, if "amount of softening agent and/or moisturizing agent is less than 20 weight %", retention of exudates in circumference of medical materials can be protected, however, as previously mentioned, in clinic an aged patient whose nutritional state is bad and metabolism activity is weakened is included, it is necessary to provide adequate safety zoon for deciding content of softening agent and/or moisturizing agent. According to pilot study like animal experiment using an animal of state of illness, there is a case that even if in the range of "amount of softening agent and/or moisturizing agent is less than 20 weight %", the animal cannot quit from edema state. Therefore, the amount of content of softening agent and/or moisturizing agent must be decided carefully.

Accordingly, although "amount of softening agent and/or moisturizing agent is less than 20 weight %" is desirable range, considering safety, less than 15 weight % is more desirable, considering further safety, less than 10 weight % is furthermore desirable, and considering very high safety for a patient of very bad state; less than 5 weight % is most desirable.

The essential point of the second invention of the present invention will be illustrated. In medical materials containing softening agent and/or moisturizing agent such as glycerin, retention of exudates, which is a side effect of the softening agent and/or moisturizing agent, can be suppressed when the content is as small as possible. However, in actual clinic, it is necessary that the content of the softening agent and/or moisturizing agent is smaller than the content of it when the product is packed and sterilized. Or, in a case of medical materials which can adjust the content of the softening agent and/or moisturizing agent to less than 20 weight % by any method, for example, by washing with solvent i.e. physiological saline, said case is within the scope of the present invention. That is, in clinic, if the content of a softening agent and/or a moisturizing agent can be lowered to less than the safety zoon by washing with solvent i.e. physiological saline, the object to suppress the retention of exudates can be accomplished also by this method.

As a method for washing, the previously mentioned "48 hours washing method" can be applied as the method to wash out completely the softening agent and/or moisturizing agent. However, in clinic, for example, in an operation room, it will be impossible to apply said "48 hours washing method". Actually, for the purpose to wash out the softening agent and/or moisturizing agent such as glycerin and to decrease the content of it to lower than the safety zoon during an operation, it is necessary to perform decreasing action of the content by washing within 30 minutes, desirably within 10 minutes. The time limit when a clinical doctor can wait to start an operation for previous arrangement, can be estimated as maximum 30 minutes, desirably within 10 minutes, therefore, it is necessary to decrease the content of softening agent and/or moisturizing agent such as glycerin to less than 20 weight % within said limit. In the present invention, this washing method is called as "30 minutes washing method". That is, washing by physiological saline within 30 minutes. By actual test, it is confirmed that glycerin content of product containing more than 40 weight % glycerin can be decreased to less than 20 weight % by washing of 10 minutes around. And as a matter of course, if the content of glycerin is decreased to less than 20 weight %, as mentioned in the essential point of the first invention of the present invention, the problem of retention of exudates which is a side effect of glycerin can be dissolved. In the present invention, the medical materials which can decrease content of softening agent and/or moisturizing agent to safety zoon, at least less than 20 weight % by above mentioned washing method, is within the scope of the present invention. This is the essential point of the second invention of the present invention.

The essential point of the third invention of the present invention will be illustrated as follows. As previously mentioned, in conventional arts, because it is necessary to provide flexibility with a bioabsorbable material, a softening agent and/or moisturizing agent are contained. And by restricting content of a softening agent less than 20 weight %, a medical material for in vivo implantation becomes hard and easily to be broken, and handling property of it deteriorates. Therefore, it is necessary to improve a bioabsorbable material itself. As a conceiving, a method to maintain flexibility by absorbing water is selected. And as a method to perform said conceiving, a method to introduce electric charge group into the bioabsorbable material, or a method to adjust hydroxyl value of the bioabsorbable material to more than 50KOH mg/g are investigated. This is the essential point of the third invention of the present invention.

The bioabsorbable material to be used in the present invention can be mentioned as follows. That is, collagen, atelocollagen, fibrous collagen, gelatine, albumin, fibrin, chitosan, chitin, carboxy methyl chitin, carboxy methyl chitosan, acethylated chitosan, acethylated chitin, chitin succinate, chitosan succinate, keratin, fibroin, hyaluronic acid, chondroitin sulfate, dextran, dextrin, cholesterol, cellulose, carboxymethyl cellulose, alginic acid, agarose, pectin, mannan, dextran, dextrin, cyclodextrin, starch, glucose, amylose, amylopectin, polymalttriosetrehalose trehalose, polyamino acid, polylycin, polypeptide, synthesized collagen, pruran, systhesized polysaccharide and derivatives thereof can be mentioned.

In the present invention, in a state that large amount of bioabsorbable material as much as possible is packed into structural gaps of porous substrate, if the content of a softening agent or moisturizing agent in the bioabsorbable material is limited to minimum amount, for the purpose to have flexibility in the level not to cause a problem of handling, the inventor investigated to provide the bioabsorbable material itself with water absorbing property. Therefore, the measure to make a bioabsorbable material to possess electric charge group is one of the essential point of the third invention of the present invention. When electric charge group such as amino group, carboxyl group, guanidyl group or sulfone group is possessed in a molecular, isoelectric point is swung to acidic side or basic side. Then these materials are solubilized at nearby isoelectric point, pushed into interstices of fibers of the porous substrate, and are put back to neutral physiological condition, these materials can be packed between interstices of fibers in state of small swelling of the bioabsorbable material.

In USP6,177,609 publication or USP6,299,693 publication a method to adjust PH from 3.5 to 3.9 for the purpose to dissolve collagen is described. Although dissolving condition is mentioned in these US Patents, these are only the mention of dissolving condition, and an idea to apply the phenomenon that the bioabsorbable material swells at putting back action from isoelectric point to physiological condition is not mentioned.

Many methods to introduce an electric charge group are already reported, and sulfonization or succinization can be mentioned as typical examples. However, in the present invention, for the purpose not to damage polyester fiber substrate by strong acid, succinization is preferably used than sulfonization. One example based on prior art which uses succinization technique in medical materials for in vivo implantation is mentioned below. After a bioabsorbable material is put into porous substrate of non-bioabsorbable, concretely into gaps of polyester fiber cloth, crosslinked and insolbilized, succinization is performed using maleate anhydride. By this method, the bioabsorbable material becomes to possess many carboxyl groups and it becomes possible to swell by absorbing water.

Further, regarding sulfonization, it is described in JPH09-510493 Publication and it can be use. Or, introduction of an electric charge group is also possible by mixing and crosslinking bioabsorbable material possessing an electric charge group such as protamine sulfonate, which is generally used as an injection.

The present invention performs succinization and introduces an electric charge group according to a method disclosed in JPS55-028947 publication. In the present invention, it is also possible to push succinyled bioabsorbable material between interstices of fibers, instead of to perform succinization after the bioabsorbable material is pushed into between gaps of fibers and crosslinked.

In a bioabsorbable material possessing electric charge group, which is recommended in the present invention, a method to put back to pH 7.2 around, which is physiological state after dissolved at closely isoelectric point can be used. This is a practical use of a phenomenon that water absorbing ratio drops mostly at isoelectric point and increases at pH 7.2 around which is physiological condition. By putting back to pH 7.2 around which is physiological condition, after pushing the dissolved bioabsorbable material at isoelectric point into interstices of fiber of porous substrate and crosslinking, the bioabsorbable material is swelled at this point and become to have flexibility, further, since volume of coating material increases protecting effect of blood leaking by coating is improved.

In the present invention, since dissolving of bioabsorbable material into solvent or washing is recommended, the bioabsorbable material becomes to be placed in the state of physiological pH 7.2 around, further, by implanting it in living body stable physiological state is formed and adequate swelling is maintained, thus, leaking of blood or other fluid, which is the purpose of coating, can be protected.

Further, as another idea to accomplish the essential point of the third invention of the present invention, there is an idea to increase the amount of hydroxyl group which bioabsorbable material possesses so as to improve water absorbency of the bioabsorbable material. In conventional arts, there is no disclosure regarding amount of hydroxyl group after crosslinking. However, in the present invention, the inventor finds out that hydroxyl group of certain amount is necessary to display effective water absorbency. Hydrogen atom in hydroxyl group is electrically charged slightly plus, on the contrary, oxygen atom is electrically charged in minus and electric attractive force acts between hydrogen atom and oxygen atom, and water molecule bonds to hydroxyl group. When large amount of hydroxyl group is contained, by so called electrochemical reaction, electric repulsive force is formed and water absorbency is improved. In the case of above mentioned material possessing electric charge group, it naturally calls in water and water absorbency is increased. Further, in cases even if a material which does not isoelectric point in acid side than pH 6.0 or alkali side than pH 9.0, amount of hydroxyl group increases by providing many hydroxyl groups, for example, by performing crosslinking with polyepoxy compound and water absorbency can be improved. In the present invention, this fact is applied.

In the present invention, the inventor of the present invention investigates what is the appropriate hydroxyl value to obtain effective water absorbing amount, by setting up the hydroxyl value of collagen as the standard. If the water absorbing amount is same as to that of collagen, it is obvious that preferable level of water absorbency can be obtained. However, since the time limit when a clinical doctor can wait to start an operation in operation room of room temperature for previous arrangement, concretely picking out a medical material for in vivo implantation from a packing and absorbing water by dipping into solvent is estimated maximum 30 minutes desirably about 10 minute, if higher water absorbency than collagen is required, consulting that hydroxyl value of collagen is about 28KOH mg/g, it is necessary to adjust hydroxyl value of the bioabsorbable material more than 50KOH mg/g. Accordingly, large amount of hydroxyl groups improves water absorbing rate, absorbs water for short time and the medical material can obtain flexibility.

To improve hydroxyl value easily, a method for crosslinking by epoxy compound mentioned below can be applied. For example, by performing chemical crosslinking by polyepoxy compound after collagen product is impregnate in fiber interstices, hydroxyl value becomes 50KOH mg/g, that is, almost 2 times of hydroxyl value of collagen can be obtained and effective water absorbency and flexibility can be obtained. When a method of polyepoxy crosslinking is used, hydroxyl value is increased and can secure water absorbency which takes in water to make a medical material for in vivo implantation flexible. Therefore, in the present invention, water absorbency and flexibility by water absorption can be provided with, by using a bioabsorbable material whose hydroxyl value is more than 50KOH mg/g.

The essential point of the fourth invention of the present invention will be illustrated as follows. This is an investigation regarding selection of a crosslinking agent. In the present invention, a method to crosslink a bioabsorbable material by afore mentioned polyepoxy resin is used as one of the measures to provide the bioabsorbable material with water absorbency or to improve water absorbency. Crosslinking by polyepoxy compound is a well known method like as a method by glutaric aldehyde or formaldehyde. However, since crosslinking intensity obtained by glutaric aldehyde crosslinking method cannot be obtained by polyepoxy compound crosslinking method, this method is not generally used in medical field. In the present invention, weak point of intensity is improved. That is, leave mechanical intensity of the medical material for in vivo implantation on a non-bioabsorbable porous substrate, and use polyepoxy compound only for crosslinking of a bioabsorbable material. Further, characteristic of water absorbency provided by increase of hydroxyl value caused in crosslinking reaction of polyepoxy compound is paid attention, and found out a fact that the bioabsorbable material can obtain flexibility after crosslinking can be actually used. Therefore, this method is used as a method to provide with flexibility to the bioabsorbable material and successes to decrease the using amount of glycerin. By a cross linking method using glutaric aldehyde or formaldehyde which is a conventional art, amino groups in the bioabsorbable material are consumed at every reaction and hydrophilicity ratio of the bioabsorbable material is deteriorated, however, since by crosslinking with polyepoxy compound, hydroxyl value increases at every reaction, hydrophilicity of the bioabsorbable material is improved or hydrophilicity by amino group replaces with hydrophilicity by hydroxyl group. From the view point that this method provides flexibility with the bioabsorbable material or maintain flexibility, this method can be said as a new method.

There are many kinds of polyepoxy crosslinking agent, however, it is desirable to be at least one selected from the group consisting of Ethylene Polyethylene Glycol Diglycidyl Ether, Polypylene Polypropylene Glycol Diglycidyl Ether, Neopentyl Glycol Diglycidyl Ether, 1,6-Hexanediol Diglycidyl Ether, Glycerol Polyglycidyl Ether, Dibromo Neopentyl Glycol Diglycidyl Ether, O-Phtalic Acid Diglycidyl Ester, Trimethylolpropane polyglycidyl Ether, Diglycerol polyglycidyl Ether, Polyglycerol Polyglycidyl Ether or SorbitolpolyglycidylEther.

While, it is not necessarily to be crosslinking by polyepoxy compound, if a bioabsorbable material can obtain total water absorbency by effects of other methods such as isoelectric point, hydroxyl group or electric charge group, using chemical crosslinking by glutaraldehyde, formaldehyde or hexamethylenediisocyanate, or crosslinking using physical energy such as heat, gamma-ray or electron beam, which are conventional arts. However, if a crosslinking method by polyepoxy compound is used, water absorbency is certainly improved and a medical material can easily obtain flexibility.

The essential point of the fifth invention of the present invention will be illustrated as follows. This is an investigation of a non-bioabsorbable porous substrate. Improvement of a non-bioabsorbable porous substrate is performed as follows. Generally, fiber material is used as a material of a non-bioabsorbable porous substrate. As starting materials, fiber substrate, for example, polyester such as polysthyleleterephthalate or polybutyelene -terephthalate, polyamide, polyolefin such as polypropylene or polyfluoro -ethylene are used. In the present invention, use of these fiber substrates is recommended, and is not adhered to how to weave or how to knit these fibers. However, regarding thickness of fiber and interstices of fibers, for the purpose to maintain flexibility of the substrate, following investigation is performed.

Fiber substrate of prior arts is constructed by fiber of normal thickness of 1.2 dtex around. A substrate made of fiber of said thickness has a problem of hardening when interstices of fibers become narrow. In the present invention, for the purpose to decrease amount of softening agent, the inventor considered it is necessary that a non-bioabsorbable porous substrate has flexibility by itself. The inventor introduced very fine fibers into structure of the substrate so as to cause slipping effect of fine fibers each other and to provide with flexibility to the non-bioabsorbable porous substrate.

Regarding introduction of very fine fibers, the art disclosed in JP2005-124959 publication filed by the inventor of the present invention can be mentioned. This art is intending to provide a medical material using very fine fibers of less than 0.5 dtex thickness and more than 4g/dtex intensity, and the art reports that a structural condition which contains more than 5% of very fine fibers of less than 0.5 dtex thickness to the total numbers of fibers is desirable. However, if these very fine fibers are simply used, fibers forms the closest packing state and cannot display flexibility. In the present invention, the inventor investigates said state more in detail and finds out a special phenomenon that by performing sufficient raising treatment to the fine fibers, openings are formed in the interstices of fibers, the openings display slipping effect of fibers and a substrate becomes flexible. And in the present invention, this method is applied positively. As disclosed in JP2005-124959 publication, numbers of very fine fibers is desirably more than 5% to the total numbers of fibers and much more is further desirable. 100%, that is, the state that all fibers are very fine fibers can be acceptable if mechanical intensity of the medical material can be maintained. In this case, it is an indispensable condition that each fiber is not in state of closest packing. Required mechanical intensity differs in cases where the medical material is applied. Therefore, in a case when stronger mechanical intensity is required, it is preferable to increase mixing ratio of fibers of normal thickness. However, in a case where fibers are used as a non-bioabsorbable porous substrate, by together use of very fine fibers with normal fibers not in state of closest packing, slipping effect between fibers can be displayed and the substrate becomes flexible. Therefore, in a medical material which becomes hard when content of a softening agent and/or a moisturizing agent becomes lower than 20 weight %, it is necessary to mix more than 5% of very fine fibers of less than 0.5 dtex to total number of fibers by raised state, that is, balky state and can provide with flexibility to the substrate.

And, together use of very fine fibers is further effective to maintain spaces between fibers of a bioabsorbable material. The state that fibers are not in closest packing state means that interstices of fibers are secured, it is possible to maintain a bioabsorbable material, which becomes brittle and breakable by water absorbing, stable in the substrate.

Regarding measurement of numbers, width of interstices of fibers of very fine fibers, following method is used. That is, non-bioabsorbable porous substrate is included by a resin for tissue fragment preparation (Technovit 7,100, kulzer & Co. GmbH, Friedrichsdorf, Germany) and sliced to a fragment of 3-5 micron thickness by a microtone with a glass knife. A picture of the fragment is taken by an optical microscope at five positions, and numbers of fibers and thickness of fiber are measured at said five points and averaged. In a case of very fine fibers of less than 0.5 dtex, cross sectional diameter of the fiber is less than 6 micron. While, since the thickness of normal fiber is more than 16 micron, discrimination and measurement of the fine fiber are easy by 200 magnification observation of an optical microscope. At the observation, inspection whether the very fine fibers become a state of closest packing or not can be done at the same time. In a case when at least one part of very fine fibers bundle is broken up and a space same as or thicker than thickness of a fiber in the interstices of fibers is observed by the observation with an optical microscope, it can be judged that the fine fibers are not in a state of closest packing. By performing above mentioned condition, slipping phenomenon between fibers is displayed and flexibility of substrate can be performed.

The essential point of the sixth invention of the present invention will be illustrated as follows. In a medical material for in vivo implantation comprising a bioabsorbable material containing a softening agent and/or a moisturizing agent and non-bioabsorbable porous substrate, in a case when content of said softening agent and/or moisturizing agent is more than 20 weight%, this invention relates to a method to adjust the weight% of the softening agent and/or moisturizing agent to less than 20 weight%. By using this adjusting method, it becomes possible to protect retention of exudates.

As previously mentioned, in a medical material for in vivo implantation comprising bioabsorbable material containing a softening agent and/or a moisturizing agent and non-bioabsorbable porous substrate of conventional art, fixed view point regarding content of it is not reported yet. Therefore, it is conjectured that glycerin content which provides with adequate flexibility and good handling property to a medical material for in vivo implantation is applied. In the present invention, for the purpose to protect retention of exudates, when a medical material for in vivo implantation contains excessive amount of glycerin, a method to decrease amount of glycerin from the medical material before or in clinic. The essential point of this method is to adjust the content of a softening agent and/or a moisturizing agent such as glycerin to less than 20 weight %.

In a case when it becomes clear that the content of a softening agent and/or a moisturizing agent such as glycerin is excessively high, it is possible to remove the softening agent and/or moisturizing agent such as glycerin almost completely by applying afore mentioned "48 hours washing method". The inventor of the present invention performed washing experiments by "48 hours washing method" on all purchasable products of medical material for in vivo implantation comprising a bioabsorbable material containing softening agent and/or moisturizing agent such as glycerin and a non-bioabsorbable porous substrate which are on the market. According to the experimental results, it is confirmed that removal of softening agent and/or moisturizing agent such as glycerin is possible by "48 hours washing method". Therefore, use of "48 hours washing method" is confirmed as one of the methods.

However, since "48 hours washing method" takes too long time and very serious endeavor is necessary to keep cleanliness of medical material during washing work, in the present invention a convenient washing method, which can be used in an operation room, is recommended. As afore mentioned, since the time limit when a clinical doctor can wait to start an operation for previous arrangement is estimated as maximum 30 minutes, desirably within 10 minutes, the present invention provides with a recommendable method to decrease the content of softening agent and/or moisturizing agent such as glycerin to less than 20 weight %. In the present invention, this washing method is called as "30 minutes washing method". Concretely, this method is a washing method of a medical material by solvent such as physiological saline within 30 minutes. In a case of artificial blood vessel containing more than 40 weight % glycerin, it is confirmed that the content of glycerin can be decreased to less than 20 weight % by "30 minutes washing method", specifically washing of about 10 minutes. And, as mentioned in the essential point of the first invention of the present invention, when content of glycerin decreases to less than 20 weight %, a problem of retention of exudates which is a side effect of glycerin becomes possible to be protected.

The essential point of the seventh invention of the present invention will be illustrated as follows. As afore mentioned, the problem of retention of exudates cannot be protected, if the content of softening agent and/or moisturizing agent is not decreased to less than 20 weight %. Therefore, many investigations become necessary. However, it is very difficult to push high hydrous bioabsorbable material, which is developed under above mentioned conception, into porous structure of fibrous non-bioabsorbable porous substrate whose spaces between fibers are narrow. Especially, for pushing fluid, in which fully swelled bioabsorbable material is dissolved, into spaces between hydrophobic fibers such as polyester fiber accompanies difficulty, and this tendency becomes more remarkable when spaces between fibers becomes narrower.

In the present invention, a method for preparation of medical material which is improved intending to push bioabsorbable material into structure of non-bioabsorbable porous substrate easily is indicated. The method will be specifically illustrated as follows. In a bioabsorbable material which is recommended in the present invention, since the recommended bioabsorbable material are characterized by having electric charge group, having more than 50KOH mg/g hydroxyl value or by crosslinked with epoxy compound, said bioabsorbable material are in high water absorbing state by not containing glycerin. By a process to push above mentioned bioabsorbable material into narrow spaces of hydrophobic fibers of non-bioabsorbable porous substrate, only small amount of the bioabsorbable material is pushed into the non-bioabsorbable porous substrate. Accordingly, in the present invention, following method is developed. That is, after closing the swelling state of a bioabsorbable material temporary by mixing with salt or alcohol, pushing the bioabsorbable material into spaces between fibers of a non-bioabsorbable porous substrate, then by removing salt or alcohol, necessary amount of bioabsorbable material can be effectively maintained in the non-bioabsorbable porous substrate. This is the essential point of the seventh invention of the present invention. By said improvement, it become possible to push necessary amount of bioabsorbable material into spaces of fiber. And, since salt or alcohol is removed at spaces between fibers, the bioabsorbable material swells at the point, accordingly the bioabsorbable material is tightly hold in spaces between fibers and becomes difficult to go out from the spaces. That is, the method has obviously advantage to fix the bioabsorbable material.

Regarding a kind of alcohol to be used, alcohol which has dehydration property and not cytotoxic in living body is desirable, and alcohol which can be removed easily at the preparation process, the alcohol also can be used. For example, it is possible to use isopropanol or methanol. In general, methanol can be used tolerably. And the recommendable region of concentration of alcohol is about 1-50 %.

Regarding a kind of salt, it is desirable too to select a salt which is not cytotoxic in living body. Further, a salt which can be removed in preparation process is desirable. Generally, use of simple table salt is recommendable. Regarding the concentration, region of 0.1-50%, desirably 5-30%, further desirably 20-30 % is recommendable.

The inventor of the present invention found out following phenomenon. That is, even if a solution in which hydrophilic material is dissolved by high concentration and gelled, viscosity drops when the concentration of salt becomes high. Concrete example will be illustrated. When gelatin is heated and dissolved, it becomes easily gelled state. By adding salt gradually to the heating process of gelatin, at the point where the concentration of salt exceeds 20%, viscosity drops and becomes smooth. Accordingly, by pressure pushing gelatin under this state into three dimensional structure of a non-bioabsorbable porous substrate, gelatin can be pushed into three dimensional structure of the non-bioabsorbable porous substrate by high concentration. After that, salt concentration is decreased by soaking into distilled water, and by removing salt, gelatin swells in three dimensional structure and do not go out from the structure. By crosslinking at this stage, the medical material for in vivo implantation comprising bioabsorbable material and non-bioabsorbable porous substrate can be prepared easily.

As a method to push high hydrous bioabsorbable material effectively into three dimensional structure of non-bioabsorbable porous substrate, a method to use isoelectric point of the bioabsorbable material is found out by the inventor of the present invention. That is, when a bioabsorbable material possessing electric charge group is dissolved, near the isoelectric point is most hard region for dissolving and has a tendency to be precipitated. Accordingly, the inventor of the present invention used practically this phenomenon. Specifically, in a case of succinized atelocollagen, isoelectric point is approximately pH 3.5. Solution of atelocollagen is prepared and adjust hydrogen ion concentration to isoelectric point gradually, succinized atelocollagen precipitates and the solution become white and muddy. By pressure pushing the solution under this state into three dimensional structure of a non-bioabsorbable porous substrate, necessary amount of succinized atelocollagen can be pushed into three dimensional structure of the non-bioabsorbable porous substrate by high concentration. After that, when hydrogen ion concentration is adjusted to neutral, succinized atelocollagen swells in three dimensional structure and do not go out from the structure. By crosslinking at this stage, the medical material for in vivo implantation comprising a bioabsorbable material and a non-bioabsorbable porous substrate can be prepared easily.

In a process to enmesh swelled bioabsorbable material into three dimensional structure of a non-bioabsorbable porous substrate, if the bioabsorbable material is swelled excessively, it is difficult to enmesh necessary amount effectively, however, if enmeshing by above mentioned method is possible, it is advantageous. The fact that the bioabsorbable material has hydrous property means that the bioabsorbable material swells by water absorption in inside of three dimensional structure of the non-bioabsorbable porous substrate. This fact means not only that the medical material obtains flexibility, but also provides with new advantage to the medical material. That is, by use of small amount of bioabsorbable material, volume of the bioabsorbable material increases by hydrous swelling and said bioabsorbable material occupies inside of three dimensional structure of the non-bioabsorbable porous substrate, and accordingly tight sealing can be performed. And in a case when the medical material is used as an artificial blood vessel or as a cardiac wall patch which contacts blood, an effect to protect leaking of blood can be performed. That is, sealing state by water swelling can be obtained.

If above mentioned essential points of the present invention are displayed, a problem of retention of exudates causes surrounding medical material can be dissolved. The medical material for in vivo implantation prepared as above can be used as a substitute or a part of tubular viscus or tubular tissue as an artificial blood vessel or an artificial trachea, further can be used by patch state to an organ or a tissue in living body as an artificial pericardium, an artificial cardiac wall, an artificial chest wall, an artificial blood vessel wall, an artificial peritoneum, an artificial dura mater or an artificial urinary bladder wall, and provide with a hybrid medical material for in vivo implantation comprising a bioabsorbable material and a non-bioabsorbable porous substrate.

### EXAMPLES

The present invention will be illustrated specifically according to Examples, however, the scope of the present invention is not intending to be restricted to these Examples.

### Example 1

As a substrate, an artificial blood vessel (Micron, water permeability ratio is 1200ml, inner diameter is 8mm, length is approximately 6cm) made of polyester fiber which is a product of Inter Vascular Ltd. (U.S.A) is used, and as a crosslinking agent, polyepoxy compound (EX810 Ethylene Glycol Diglycidyl Ether) which is a product of Nagase Kasei Co., Ltd., is used. The crosslinking agent is dissolved in ethanol so as the concentration to be 3%, and as a bioabsorbable material, atelocollagen is used. Specimen prepared by coating atelocollagen on the substrate is lyophilized and reacted in ethanol solution containing the crosslinking agent at room temperature for 24 hours. Hereinafter, bioabsorbable material can be shortened simply to coating material, and artificial blood vessel coated with bioabsorbable material can be shortened to coated artificial blood vessel.

Average weight of an artificial blood vessel composed of polyester fiber alone before coating is 0.3905g, and average weight after coated by atelocollagen and crosslinked is 0.6287g by completely dried state. That is, weight of coating material including crosslinking agent is 0.2382g. In this dried condition, the artificial blood vessel is hardened and can be broken easily.

As a softening agent and/or a moisturizing agent, glycerin which has both properties of these two agents is selected, and prescribed weight of glycerin is impregnated to the artificial blood vessel coated by atelocolagen. According to previous basal experiments, it becomes clear that other polyvalent alcohol, for example, xylitol, sorbitol or mannitol have also both properties of these two agents, and by impregnating large amount of these compounds, complication of retention of exudates is caused at surroundings. In the present invention, glycerin which is most frequently used in actual clinic is selected.

Concretely, 1% glycerin solution, 5% glycerin solution, 10% glycerin solution, 20% glycerin solution and 30% glycerin solution, which are diluted by ethanol, are prepared and a method to make sink these solution into the artificial blood vessel and to dry up is applied. And said operation is repeatedly performed.

The average amount that the artificial blood vessel of inner diameter 8mm, length approximately 6cm can include alcohol solution containing glycerin at one time is approximately 0.30g, therefore, in a case of 1% glycerin solution, the artificial blood vessel can contain 0.003g of glycerin at one time sinking. In a case of 5% glycerin solution 5 times amount of glycerin, in a case of 10% glycerin solution 10 times amount of glycerin, in a case of 20% glycerin solution 20 times amount of glycerin, in a case of 30% glycerin solution 30 times amount of glycerin and in a case of 50% glycerin solution 50 times amount of glycerin can be contained by the artificial blood vessel. By applying this method, properly use of glycerin solutions of different concentration and altering of times to sink into artificial blood vessel, adjusting of glycerin containg amount becomes possible.

Accordingly, amount of glycerin to be impregnated is prescribed as follows. That is, since total weight of artificial blood vessel, coating material and crosslinking agent is 0.6287g, for the purpose to obtain 20% glycerin containing state, 0.1572g of glycerin must be contained. By sinking 50% glycerin solution for one time, 0.15g of glycerin can be contained, and by sinking 1% glycerin solution for three times, further 0.009g of glycerin can be contained, accordingly, it may be possible to prepare an artificial blood vessel of 20% glycerin concentration by this operation. By actual experiment, weight of the artificial blood vessel is increased to 0.1578g, and an artificial blood vessel of about 20% glycerin concentration can be obtained. By this method, an artificial blood vessel of 30% glycerin concentration, an artificial blood vessel of 25% glycerin concentration, an artificial blood vessel of 20% glycerin concentration, an artificial blood vessel of 15% glycerin concentration, an artificial blood vessel of 10% glycerin concentration, an artificial blood vessel of 5% glycerin concentration and an artificial blood vessel of 0% glycerin concentration (namely an artificial blood vessel not containing glycerin) are prepared. 3 specimens of each level are prepared, and these specimens are sterilized with gas (ethylene oxide gas). Among above specimens, specimens whose glycerin concentration is more than 20 weight % have almost same flexibility as an artificial blood vessel used in actual clinic, however, specimens whose glycerin concentration is less than 20 weight % become hard, and a specimen of 0% glycerin concentration is hard and is easily broken by pinching with a tweezers.

Then, in clean condition, approximately 5cm of descending aorta is resected from a beagle dog under general anesthesia, and a prepared artificial blood vessel of 5cm length is implanted to the resected part and observed after 2 weeks. According to the observation results, in cases of artificial blood vessel of 30% and 25% glycerin concentration, retention of exudates is caused in a circumference of all artificial blood vessels and a capsule which surrounds the exuded liquid is formed. While, in cases of artificial blood vessel of 20%, 15%, 10%, 5% and 0% glycerin concentration, retention of exudates cannot be observed. These are results obtained by 3 specimens implantation, and said phenomena are observed in all experiments. From these experiments, it becomes clear that when amount of glycerin is less than 20 weight % to total weight of an artificial blood vessel, retention of exudates does not cause in circumference of all artificial blood vessels.

In a clinical report, there is a report informing that even after one month from implantation of an artificial blood vessel, retention of exudates can be observed in a circumference of the artificial blood vessel. However, in the present invention, considering that retention of exudates is gradually absorbed, observation term is set up to two weeks after operation in animal experiment. As obviously understood from experimental results, state of surrounding tissue and degree of retention of exudates are different according to difference of amount of glycerin content.

### Example 2

From the results of Example 1, it becomes clear that retention of exudates in circumference of medical material for in vivo implantation is influenced by amount of glycerin. Further, since it becomes clear that there is a border between 25% and 20%, re-estimation experiments to investigate accurate amount of glycerin are performed. Specimens of artificial blood vessel are prepared by same method as mentioned in Example 1. That is, an artificial blood vessel (Micron, water permeability ratio 1200ml, inner diameter 8mm, length approximately 6cm) made of polyester fiber which is a product of Inter Vascular Ltd. (U.S.A) is used, and as a crosslinking agent, polyepoxy compound (EX810 Ethylene Polyethylene Glycol Diglycidyl Ether) which is a product of Nagase Kasei Co., Ltd., is used. The crosslinking agent is dissolved in ethanol so as the concentration to be 3%, specimen prepared by coating atelocollagen on the substrate is lyophilized and reacted in ethanol solution containing the crosslinking agent at room temperature for 24 hours. Average weight of after atelocollagen coating under completely dried state is 0.6287g. In this state, different amount of glycerin is impregnated. As a method to impregnate glycerin, the method mentioned in Example 1 is applied. That is, 1% glycerin solution, 5% glycerin solution, 10% glycerin solution, 20% glycerin solution and 30% glycerin solution, which are diluted by ethanol are prepared and a method to make sink these solution into the artificial blood vessel and dry up and to repeat said operations is applied. By this method, an artificial blood vessel of 24% glycerin concentration, an artificial blood vessel of 23% glycerin concentration, an artificial blood vessel of 22% glycerin concentration, an artificial blood vessel of 21% glycerin concentration are prepared. Three specimens for each level are prepared, and these specimens are sterilized with gas.

Then, approximately 5cm of descending aorta is resected from a beagle dog under general anesthesia, and a prepared artificial blood vessel of 5cm length is implanted to the resected part and observed after 2 weeks. According to the observation results, in cases of artificial blood vessel of 24%, 23% and 22% glycerin concentration, retention of exudates is observed in a circumference of all artificial blood vessels, and in case of artificial blood vessel of 21%, tissue surrounding the artificial blood vessel becomes to form edema state. Considering that the edema state is the state that exuded liquid permeates into tissue, it will be possible to protect retention of exudates when amount of glycerin is less than 20 weight %.

### Example 3

By same method as to Examples 1 and 2, a specimen is prepared using an artificial blood vessel (Micron, water permeability ratio 1200ml, inner diameter 8mm, length approximately 6cm) made of polyester fiber which is a product of Inter Vascular Ltd. (USA). As a bioabsorbable material, carboxymethyl chitosan is used. As a crosslinking agent, polyepoxy compound EX313 Glycerol Polyglycidyl Ether, which is a product of Nagase Kasei Co., Ltd., is used.

Average weight of an artificial blood vessel made of polyester before coating is 0.3099g, and weight of the artificial blood vessel after coating of carboxymethyl chitosan is 0.7564g under complete dried condition. That is, weight of coating material containing crosslinking agent is 0.4465g by average, and in this dry condition, the artificial blood vessel is in a state of hardened and easily to be broken. Then, glycerin is impregnated in this state. As a method to impregnate glycerin, the method mentioned in Example 1 is applied. In a case of this artificial blood vessel, since amount of alcohol solution which can be contained at one time is about 0.40g by average, in a case of 1% glycerin solution, the artificial blood vessel contains 0.004g of glycerin at one time. According to above mentioned thinking, amount of glycerin is prescribed same as to Examples 1 or 2, three specimens of following five kinds of artificial blood vessel, that is, containing 23% glycerin, 22% glycerin, 21% glycerin, 20% glycerin and 19% glycerin to the total weight are prepared and sterilized with gas.

Then, approximately 5cm of descending aorta is resected from a beagle dog under general anesthesia, and a prepared artificial blood vessel of 5cm length is implanted to the resected part and observed after 2 weeks. According to the observation results, in cases of artificial blood vessel of 23% and 22% glycerin concentration, retention of exudates is observed in a circumference of all artificial blood vessels, while, in a case of artificial blood vessel of 21% glycerin concentration, tissue surrounding the artificial blood vessel becomes to form edema state, further in cases of artificial blood vessel of 20% and 19% glycerin concentration, retention of exudates is not observed. These results are commonly obtained by three implanted specimens. Considering these experimental results, it becomes clear that retention of exudates causes in circumference of artificial blood vessel can be protected by decreasing the amount of glycerin to less than 20 weight %. This result is same as to the results obtained in Examples 1 or 2, therefore, it becomes clear that, even if the kinds of coating material is changed, the retention of exudates in circumference of artificial blood vessel is not changed, but the degree of retention of exudates is changed by difference of glycerin content.

### Example 4

Same as to Example 3, three specimens of following five kinds of artificial blood vessel, that is, containing 23% glycerin, 22% glycerin, 21% glycerin, 20% glycerin and 19% glycerin to the total weight are prepared, and 5mm length of fragment is cut off from each specimen and ring shape fragment is prepared.

These are sterilized .with gas, and under clean condition, said ring shape specimen is inserted under dorsal subcutaneous tissue of a rat maintaining a ring state. One specimen is inserted for each one rat, and three rats are used for one kind of specimen, accordingly, 15 rats are used in total. After one week, dorsal of every rats are incised under general anesthesia and observed. In cases of ring shape artificial blood vessel fragments of 23% and 22% glycerin content, retention of exudates are observed in inner side and outer side of all rings, and thin capsule is formed surrounding the retention of exudates, however, migration of cells such as fibroblast which regenerates tissue is not observed. In a case of artificial blood vessel of 21% glycerin content, migration of young connective tissue is observed in inner side of ring and the young connective tissue forms an edema, however, retention of exudates and a capsule surrounding the retention of exudates are not observed. On outer side of the artificial blood vessel, young connective tissue is stuck and the tissue is in state of edema. In the meanwhile, in cases of artificial blood vessel of 20% and 19% glycerin content, tissue of edema state and exuded liquid are not observed on outer side and inner side of ring of artificial blood vessel specimen and only sticking of connective tissue is observed. These results are obtained in all three implanted specimen.

According to these results, for the purpose to observe retention of exudates which is the influence of contained glycerin, it is understood that the animal experiment using dorsal subcutaneous tissue of rat can replace the animal experiment of artificial blood vessel implantation using a beagle dog, and by this animal experiment by a rat can shorten the observation term. That is, it is not necessary to perform an animal experiment to implant an artificial blood vessel into descending aorta of a beagle dog. Further, it is confirmed that when amount of glycerin is less than 20 weight%, retention of exudates does not cause in a circumference.

### Example 5

A coated artificial blood vessel is prepared by same method as to Example 1 except using fibrous collagen instead of atelocollagen as a coating material of a bioabsorbable material, and amount of glycerin content is changed. That is, amount of glycerin to total weight of artificial blood vessel, coating material and a crosslinking agent is changed by 15 levels, specifically, 23%, 22%, 21%, 20%, 19%, 18%, 17%, 16%, 15%, 13%, 12%, 11%, 10% and 9%. Three specimens for each level are prepared.

Same as to Example 4, 5mm length of fragment is cut off from these prepared artificial blood vessel specimen and ring shape specimen are prepared, then a ring shape specimen is inserted under dorsal subcutaneous tissue of a rat and is observed after one week. Same as to Example 4, in cases of specimens of less than 20% glycerin content, retention of exudates is not observed. Accordingly, it is proved from experimental results inserting a specimen under dorsal subcutaneous tissue of a rat that the retention of exudates is influenced by glycerin content and not influenced by kind of coating material. And, it becomes clear that when amount of glycerin is less than 20 weight % to total weight of specimen, retention of exudates, which is a side effect of glycerin, does not cause.

### Example 6

Same experiment as to Example 5 is performed except using gelatin instead of atelocollagen and same results as to Example 5 are obtained. From the results, it becomes clear that retention of exudates is influenced by glycerin content and not influenced by kind of coating material, and retention of exudates in circumference tissue is influenced by glycerin content.

### Example 7

Same experiment as to Example 5 is performed except using carboxy methyl cellulose as a coating material of bioabsorbable material. At the experiments, crosslinking by polyepoxy compound is not made but a heat crosslinking is made. The artificial blood vessel is placed in dry condition and heated at 120°C under vacuum condition for 12 hours. Carboxy methyl cellulose is insolubilized by heat crosslinking. By this treatment, the artificial blood vessel becomes hard so that not to be used in clinical spot. Then, experiments to insert a specimen under dorsal subcutaneous tissue of a rat are performed and similar results as to Example 5 are obtained. Accordingly, it becomes clear that retention of exudates is influenced by glycerin content, even if crosslinking method for coating material is changed, and when glycerin content becomes less than 20 weight%, retention of exudates which is side effect of glycerin does not cause. Further, by comparing results obtained by this experiment with results obtained by Examples 1-6, it becomes clear that by performing crosslinking reaction with epoxy compounds, flexibility which cannot be obtained by heat crosslinking, which is conventional art, can be obtained.

### Example 8

In afore mentioned Example 7, cross linking by heat is performed instead of crosslinking by epoxy compounds, and in this Example, crosslinking by glutaric aldehyde is performed. As a coating material for bioabsorbable material, gelatin is used. 3% glutaric aldehyde solution is used and crosslinked for 5 hours. After crosslinking, an artificial blood vessel is hardened. Then sterilized by gas, and experiments same as Example 7 to insert a specimen under dorsal subcutaneous tissue of a rat are performed and similar results as to Example 7 is obtained. Accordingly, it becomes clear that retention of exudates is influenced by glycerin content, even if crosslinking method for coating material is changed, medical material becomes hard, or, a coating material for bioabsorbable material is changed. Further, by comparing results obtained by this experiment with results obtained by Examples 1-6, it becomes clear that by performing crosslinking reaction with epoxy compounds, flexibility which cannot be obtained by crosslinking by glutaric aldehyde, which is conventional art, can be obtained.

### Example 9

In afore mentioned Example 7, cross linking by heat is performed instead of crosslinking by epoxy compounds, and in this Example, crosslinking by electron beam irradiation is performed. As a coating material for bioabsorbable material, chitosan is used. Deacetylinity from chitin to chitosan is 87%. After this chitosan is dissolved into acetic acid solution, coated on artificial blood vessel and dried. Then, washed by distilled water and crosslinked by electron beam of 25G intensity. By this process, the artificial blood vessel becomes hard. Since sterilization can be done by electron beam irradiation simultaneously, sterilization by gas is not made. And experiments same as Example 7 to insert the obtained specimen under dorsal subcutaneous tissue of a rat are performed and similar results as to Example 7 are obtained. Accordingly, it becomes clear that retention of exudates is influenced by glycerin content, even if crosslinking method for coating material is changed, medical material becomes hard, or, a coating material for bioabsorbable material is changed. Further, by comparing results obtained by this experiment with results obtained by Examples 1-6, it becomes clear that by performing crosslinking reaction with epoxy compounds, flexibility which cannot be obtained by electron beam crosslinking, which is conventional art, can be obtained.

### Example 10

As an Example used for region excepting cardiovascular region, Example for reparation of an artificial abdominal wall is shown. Same specimen same as an artificial blood vessel prepared in Example 2 is prepared. That is, an artificial blood vessel (Micron, water permeability ratio 1200ml, inner diameter 8mm, length approximately 6cm) made of polyester fiber which is a product of Inter Vascular Ltd. (USA) is used, and as a crosslinking agent, polyepoxy compound (EX810 Ethylene Glycol Diglycidyl Ether) which is a product of Nagase Kasei Co., Ltd., is used. To the prepared artificial blood vessel, ethanol containing glycerin is sunk, and an artificial blood vessel of 24% glycerin concentration, an artificial blood vessel of 23% glycerin concentration, an artificial blood vessel of 22% glycerin concentration, an artificial blood vessel of 21% glycerin concentration, an artificial blood vessel of 20% glycerin concentration and an artificial blood vessel of 19% glycerin concentration are prepared. Three specimens for each level are prepared, and these specimens are sterilized with gas.

Then laparotomy operation is made to a rabbit under general anesthesia and clean condition, 2.5cm × 3.5cm width is cut off as muscle layer of abdominal wall, pellicle and peritoneum in one body, and an artificial abdominal wall prepared by cut the prepared artificial blood along with long axis direction is implanted. Outer surface of the artificial abdominal wall is covered by skin and inner surface of the artificial abdominal wall is made contact with intestine. After two weeks, laparotomy operation is made on said treated rabbit again and observed. As results, in all artificial abdominal walls of 24%, 23% and 22% glycerin content, retention of exudates is observed between skin and artificial abdominal wall. In a case of 21% glycerin content, tissue in circumference of artificial abdominal wall, edema state is formed. Further, in cases of 20% and 19% glycerin content, retention of exudates or edema state cannot be observed. These phenomena are equally observed in all cases of three rabbits. Considering that the edema state is the state that exudates is permeated into tissue and retained in tissue, when glycerin content is less than 20 weight %, from these experimental results, it is considered that retention of exudates can be protected:

### Example 11

Example indicating that weight % of a softening agent and/or a moisturizing agent is important in final actual clinic is shown.

Experiments of Example 10 using a rabbit repeated reliably, except to wash abdominal cavity of the rabbit with physiological saline during laparotomy operation. Washing of abdominal cavity is generally performed process during laparotomy operation. At this washing process, the artificial abdominal wall sewed to abdominal wall contacts with physiological saline and washed at same time. After washing and just before closure process of incised wound, a small portion is cut off from the end of the artificial abdominal wall and used for measurement of glycerin content. Laparotomy operation is made again to a rabbit used for this experiment and observed after two weeks. As results, in all artificial abdominal walls of 24% glycerin content, small amount of retention of exudates is observed between skin and artificial abdominal wall. However, in cases of 23%, 22%, 21%, 20% and 19% glycerin content, retention of exudates or edema state cannot be observed. These phenomena are equally observed in all cases of three rabbits. Thereupon, glycerin content of end part of the artificial abdominal wall is measured again, and glycerin content of 24% artificial abdominal wall is decreased to 21% by average. In cases of artificial abdominal wall of 23%, 22%, 21%, 20% and 19% glycerin content, glycerin content is decreased to 17%, 15%, 16%, 12% and 7%. According to the results, it becomes clear that the glycerin content decreases finally to less than 20 weight % by washing of inside of cavity in actual clinic, and to be less than 20 weight % is important to protect complication.

### Comparative Example 1

As a typical example of collagen coated artificial blood vessel using glycerin as a softening agent and/or a moisturizing agent, Hemashield artificial blood vessel which is a product of Boston Scientific Co., Ltd., (U.S.A. New Jersey State) is used. This artificial blood vessel is coated with collagen, and the collagen is crosslinked and insolubilized by formaldehyde. As the first, glycerin content of the artificial blood vessel is measured. Three pieces of artificial blood vessel of about 6cm length, 8mm inner diameter are prepared, and dried by a lyophilizer. Average weight of these specimens is 0.7564g. Then glycerin is removed by "48 hours washing method". Specifically, these specimens are dipped in one liter of distilled water and shaken for 48 hours. Distilled water is exchanged to new one every 8 hours. At the exchange process of distilled water, the specimen of the artificial blood vessel is wrapped in gauze, and water contained in the specimen is removed by pushing it lightly. By dipping it in distilled water again, water in the artificial blood vessel wall can be exchanged. By these operations glycerin can be eluted completely from the artificial blood vessel to distilled water after 48 hours. After that, the specimen of the artificial blood vessel is dried up completely and the weight of it is measured and the averaged weight is 0.4402g. Accordingly, total weight of glycerin can be calculated from these measured value, and presumed as 0.3162g. That is, 41.8% to total weight.

### Comparative Example 2

Hemashield artificial blood vessel which is a product of Boston Scientific Co., Ltd., (U.S.A. New Jersey State) and is used in above mentioned Comparative Example 1 is used as a typical example of collagen coated artificial blood vessel using glycerin as a softening agent and/or a moisturizing agent. Said artificial blood vessel is implanted into a chest descending aorta of three beagle dogs. Size of the artificial blood vessel is 6cm length and 8mm inner diameter. The artificial blood vessel has flexibility and handling property of it is good. After 2 weeks from implantation, circumference of the artificial blood vessel is observed. Circumference surrounding the artificial blood vessel is covered with a capsule, and in the capsule exudates retains surrounding the artificial blood vessel and the exudates is oppressing the artificial blood vessel. In inner surface of the artificial blood vessel, thrombus is stuck thickly. Since this phenomenon is observed commonly to three dogs, it becomes clear that in a case of medical material for in vivo implantation whose content of glycerin is large, retention of exudates causes in circumference.

### Example 12

Six pieces of specimens of Hemashield artificial blood vessel of 6cm length and 8mm inner diameter, which is used in Comparative Example 1, are prepared. Experiments to confirm whether it is possible to protect causing of retention of exudates in circumference if amount of glycerin is decreased before implantation using "30 minutes washing method" are performed. "30 minutes washing method" of this Example is a method characterizing to dip an artificial blood vessel in 500ml solvent, specifically physiological saline or distilled water, and after light shaking, the specimen is wrapped in gauze and to remove water contained in the specimen. By said method, Hemashield artificial blood vessel is washed. Actual washing time is about 10 minutes. Average weight of dry state of six artificial blood vessel of 6cm length and 8mm inner diameter before washing is 0.7725g. And average weight of the artificial blood vessel after "washing" is 0.4877g. Further, 3 pieces of specimens among 6 pieces of specimens are washed by "48 hours washing method" with distilled water. That is, these specimens are dipped into one liter of distilled water, glycerin is removed perfectly by changing water at every 8 hours. These specimens are dried up and weights of these specimens are measured. Average weight is 0.4541g. From these values, it is calculated that this artificial blood vessel contains 41.2% of glycerin, therefore, it is calculated that glycerin content is decreased to 4.3% by "30 minutes washing method".

Three specimens which are treated only by ".30 minutes washing method" are sterilized by gas and implanted to chest descending aorta of dog. The artificial blood vessel at implantation is hard and handling property is bad, however, when dipped into physiological saline, almost similar flexibility compared with flexibility when glycerin is contained is recovered and handling property becomes good. Thus the state after 2 weeks from implantation is observed. Circumference of the artificial blood vessel is covered by young bonding tissue, and retention of exudates is not observed in surrounding, further also formation of capsule is not observed. This result is commonly observed in three dogs. From these results, it becomes clear that even if a medical material for in vivo implantation which contains large amount of glycerin can avoid side effect of glycerin by decreasing glycerin content by washing just before the actual use and finally by adjusting glycerin content to less than 20 weight %, and if flexibility in hydrous state by physiological saline is obtained, handling property does not deteriorate.

### Comparative Example 3

Same investigation as to Comparative Example 1 is performed by using Interguard artificial blood vessel, which is a product of Inter Vascular Co., Ltd. (U.S.A. Florida State), as a typical example of collagen coated artificial blood vessel being on the market which uses glycerin as a softening agent and/or a moisturizing agent. This artificial blood vessel is coated with collagen, and the collagen is crosslinked and insolubilized by glutaraldehyde. As the first, glycerin content of the artificial blood vessel is measured. Nine pieces of artificial blood vessel of about 6cm length, 8mm inner diameter are prepared, and dried by a lyophilizer and a dehumidi_fier. Average weight of these specimens is 0.4877g. Then, three pieces are selected from nine pieces. Selected three pieces are washed according to afore mentioned "washing," method and dried again, then the weight is measured. Average weight is 0.3949g. That is, 0.0928g of soluble component, namely glycerin is removed. Then another three pieces among nine pieces are selected and washed according to "48 hours washing method". That is, these three specimens are dipped in one liter of distilled water and shaken for 48 hours. Distilled water is exchanged to new one every 8 hours, and makes glycerin elute completely from the artificial blood vessel into distilled water. After that, dry weight of these three specimen is measured, and average weight is 0.3799g. That is, in this process, 0.1078g of glycerin is flown out, and this is total weight of glycerin. This weight corresponds to 22.1% of total weight.

### Comparative Example 4

Interguard artificial blood vessel which is a product of Inter Vascular Co., Ltd. (U.S.A. New Jersey State) is used as a typical example of collagen coated artificial blood vessel using glycerin as a softening agent and/or a moisturizing agent. Not washed three pieces of specimen among nine pieces of specimen are selected and implanted into a chest descending aorta of three dogs. The artificial blood vessel has flexibility and handling property is good. After 2 weeks from implantation, circumference of the artificial blood vessel is observed. Circumference surrounding the artificial blood vessel is covered with a capsule, and in the capsule small amount of exudates retains surrounding the artificial blood vessel, further young bonding tissue of edema state can be observed. From these results, it becomes clear that in a case of medical material for in vivo implantation whose content of glycerin is 22.1%, concretely artificial blood vessel, exudates retains surrounding the artificial blood vessel and a part of exudates brings a tissue on edematous state.

### Example 13

Interguard artificial blood vessel, which is used in afore mentioned Comparative Example 3, is used. Investigation to confirm whether it is possible to protect retention of exudates in circumference or not by decreasing amount of glycerin in the artificial blood vessel by washing it before implantation is performed. As shown in afore mentioned Comparative Example 2, average weight of the artificial blood vessels is 0.4877g. Then, three pieces are selected from nine pieces. Selected three pieces are washed according to afore mentioned "washing" method and dried again, then the weight is measured. Average weight is 0.3949g. That is, 0.0928g namely 19.0% of glycerin is flown out by "washing", and since total content of glycerin before "washing" is 22.1%, glycerin content after "washing" can be calculated as 3.1%.

Then, the washed artificial blood vessel is sterilized and implanted into chest descending aorta of dog. The artificial blood vessel at implantation process is hard, however, by dipping into physiological saline it becomes flexible, and almost equal flexibility to that of when glycerin is contained is recovered and handling property is good. The state after 2 weeks from implantation is observed. Circumference of the artificial blood vessel is covered with young bonding tissue, and retention of exudates is not observed in circumference, further edematous state is not observed on the bonding tissue. This result is commonly observed in three dogs. From these results, it is indicated that even if a medical material for in vivo implantation which contains large amount of glycerin can avoid a side effect of glycerin by adjusting glycerin content to less than 20 weight % by washing just before the actual use, and retention of exudates in circumference can be protected, and if flexibility in hydrous state by physiological saline is obtained, handling property does not deteriorate.

### Comparative Example 5

Same investigation as to Comparative Example 3 is performed by using Gelweave artificial blood vessel, which is a product of Vascutek Co., Ltd. (Scotland), as a typical example of gelatin coated artificial blood vessel being on the market which uses glycerin as a softening agent and/or a moisturizing agent. This artificial blood vessel is coated with gelatin, and the gelatin is crosslinked and insolubilized by formaldehyde. As the first, glycerin content of the artificial blood vessel is measured. Nine pieces of artificial blood vessel of about 6cm length, 8mm inner diameter are prepared, and dried by a lyophilizer. Average weight of these specimen is 0.5680g. Then, three pieces are selected from nine pieces. Selected three pieces are washed according to afore mentioned "washing" method and dried again, then the weight is measured. Average weight is 0.4049g. That is, 0.1586g of soluble component, namely glycerin is removed. Then another three pieces among nine pieces are selected and washed according to "48 hours washing method". That is, these three specimens are dipped in one liter of distilled water and shaken for 48 hours. Distilled water is exchanged to new one every 8 hours, and makes glycerin elute completely from the artificial blood vessel into distilled water. After that, dry weight of these three specimens is measured, and average weight is 0.3995g. That is, in this process, 0.1685g of glycerin is flown out, and if whole glycerin is flown out by said washing, total weight of glycerin is 0.1685g, which is 29.7% to total weight 0.5687g.

### Comparative Example 6

Among nine pieces of Gelweave artificial blood vessel used in Comparative Example 3, not washed three pieces of specimen are selected and implanted into a chest descending aorta of three beagle dogs. The artificial blood vessel has flexibility and handling property is good. After 2 weeks from implantation, circumference of the artificial blood vessel is observed. Circumference surrounding the artificial blood vessel is covered with a capsule, and in the capsule small amount of exudates retains. This result is commonly observed in three dogs. From these results, it becomes clear that in a case of medical material for in vivo implantation whose content of glycerin is 29.7%, concretely artificial blood vessel, exudates retains surrounding the artificial blood vessel.

### Example 14

Gelweave artificial blood vessel, which is used in afore mentioned Comparative Example 5, is used. Investigation to confirm whether it is possible to protect retention of exudates in circumference by decreasing amount of glycerin in the artificial blood vessel by washing it before implantation is performed. As shown in afore mentioned Comparative Example 5, average weight of the artificial blood vessels is 0.5680g. Then, three pieces are selected from nine pieces. Selected three pieces are washed according to afore mentioned "washing" method and dried completely again, then the weight is measured. Average weight is 0.4049g. That is, 0.1586g namely 27.9% of glycerin is flown out by "washing", and since total content of glycerin before "washing" is 29.7%, glycerin content after "washing" can be calculated as 1.8%.

Then, the washed artificial blood vessel is sterilized and implanted into chest descending aorta of a dog. The artificial blood vessel at implantation process is hard, however, by dipping into physiological saline it becomes flexible and handling property is good. The state after 2 weeks from implantation is observed. Circumference of the artificial blood vessel is covered with young bonding tissue, and retention of exudates is not observed in circumference, further edematous state is not observed on the bonding tissue. This result is commonly observed in three dogs. From these results, it is indicated that even if a medical material for in vivo implantation which contains large amount of glycerin and causes retention of exudates, which is side effect of glycerin, can avoid the side effect of glycerin by adjusting glycerin content to less than 20 weight % by washing just before the actual use, and retention of exudates in circumference can be protected, and if flexibility in hydrous state by physiological saline is obtained, handling property does not deteriorate.

### Example 15

In afore mentioned Example1, in a case when glycerin content is zero, that is, a softening agent and/or a moisturizing agent is not contained, detail of estimation will be explained. As afore mentioned, in Example 1 which coats atelocollagen or in Example 2 which coats carboxymethyl chitosan, when glycerin is mixed with alcohol and sunk, specimen gradually becomes flexible. However, in control in which glycerin is not sunk, the specimen is hard and is easily broken further handling property is very bad. Therefore, the specimen is easily broken by only pinching with tweezers. Accordingly, in a case to take out the specimen from a sterilizing package, it is necessary to sprinkle physiological saline and to wait about 3 minutes. And, the specimen sucks physiological saline up and becomes flexible, thus it becomes not to be broken even if pinched with tweezers. When the specimen is hold by fingers of an operator, hardness that the operator feels difficulty for operation does not come out, and the specimen has similar flexibility with a specimen which contains more than 20 weight of glycerin. Accordingly, safe implantation of it into chest descending aorta of a dog becomes possible. And, handling property during operation is good. From said results, it becomes clear that in a specimen whose glycerin content is zero, flexibility can be provides with by sinking solvent such as physiological saline into the specimen before use.

In the meanwhile, as explained in Example1, in a case of specimen whose content of glycerin is less than 20 weight % or a specimen whose content of glycerin is decreased as small as possible finally to zero, retention of exudates is not observed in circumference of an artificial blood vessel. Therefore, even if the specimen which does not contain a softening agent and/or a moisturizing agent, it becomes flexible by being hydrous state and there is no problem regarding handling property, accordingly it becomes clear that the retention of exudates which is an object of the present invention can be protected.

### Example 16

Example proving to obtain flexibility by hydrous state will be shown. According to same method to Example 1, an artificial blood vessel coated by atelocollagen and according to Example 2, an artificial blood vessel coated by carboxymethyl chitosan are prepared and crosslinked by epoxy compound EX-810. For the purpose to provide with hydrous property, trehalose, which is a typical moisturizing agent, is contained in atelocollagen and carboxymethyl chitosan by 1% ratio. Physical property does not change by adding of trehalose. And, in dry condition after crosslinking reaction, it is same as the control of Example-1 which does not contain trehalose. The prepared artificial blood vessels are sterilized then touched, but they are state of hard and easy to be broken. Therefore, physiological saline is sprinkled. The artificial blood vessels absorb physiological saline rapidly and become flexible by one minute, and can obtain handling property to be used in operation. As mentioned above, by providing with hydrous property by containing a moisturizing agent such as trehalose by less than 20 weight %, it is possible to maintain flexibility of usable level only by contacting with physiological saline.

### Example 17

Specimen prepared in afore mentioned Example 13 is cut off to 5mm length and prepared as a ring shape specimen and sterilized by EOG gas. Then the specimen is inserted under dorsal subcutaneous tissue of a rat as mentioned in Example 4 and after one week the specimen is observed. According to the observation results, retention of exudates in circumference are not observed in all specimens. These results indicates that by containing a moisturizing agent such as trehalose, not a softening agent and/or a moisturizing agent such as glycerin, by about 1%, that is, less than 20 weight %, an artificial blood vessel can obtain flexibility only by contacting with physiological saline, further does not cause of handling problem, and does not cause retention of exudates when implanted in a living body.

### Example 18

Example which proves that a medical material can obtain flexibility by being hydrous state will be shown. In this Example, kind of moisturizing agent is changed. In Example 16, trehalose is used as a typical moisturizing agent, and in this Example, xylitol is used. Amount of xylitol used in this Example is 1%, which is same amount to trehalose used in Example 16. And same specimens as Example 16 are prepared. Obtained results indicate that water absorbing rate is slightly slow than the specimen which uses trehalose, however, all specimens become flexible by 30 seconds after sprinkling of physiological saline. From these results, it becomes clear that by use of a moisturizing agent instead of large amount of glycerin which is a softening agent and/or a moisturizing agent, flexibility can be obtained and no problem is caused in handling at operation.

### Example 19

Example which proves that a medical material can obtain flexibility by being hydrous state will be shown. In this Example, kind of moisturizing agent is changed. In Example 16, trehalose is used as a typical moisturizing agent, and in this Example, sorbitol is used. Amount of sorbitol used in this Example is 1%, which is same amount to trehalose used in Example 16. And same specimens as Example 16 are prepare, and similar results are obtained as to the case when xylitol is used as a moisturizing agent. These results make it clear that even if kind of moisturizing agent is changed, by using adequate moisturizing agent instead of large amount of glycerin which is a softening agent and/or a moisturizing agent, flexibility can be obtained and no problem is caused in handling at operation. That is, even if content of a softening agent and/or a moisturizing agent is less than 20 weight %, flexibility can be obtained by such a different means.

### Example 20

Example indicates that a medical material can be hydrous state when a bioabsorbable material has electric charge group and isoelectric point is acid side than pH6.0 or basic side than pH9.0. Atelocollagen coated artificial blood vessel is prepared by same method to Example 1, and crosslinked with epoxy compound EX-810. Then, the specimen is succinized by maleic anhydride. Succinization method is followed to the method disclosed in JPS55-028947 publication. Isoelectric point of the prepared succinized atelocollagen is pH4.0. The obtained specimen is dried by a lyophilizer and sterilized by gas. The specimen is hardened and is in state of easy to be broker. Then, physiological saline is sprinkled on the specimen, and the specimen absorbs physiological saline by 40 seconds, swelled and become flexible. The obtained flexibility is almost same or more flexible compared to that of a specimen which uses a softening agent and/or a moisturizing agent. These results make it clear that by making a bioabsorbable material possesses electric charge group, a specimen obtains flexibility by being hydrous state and does not cause problem in handling property, even if glycerin which is a softening agent and/or a moisturizing agent is not used. That is, even If content of a softening agent and/or a moisturizing agent is less than 20 weight %, flexibility can be obtained by such a different means.

### Example 21

The specimen prepared in afore mentioned Example 20 and estimated in dorsal subcutaneous tissue of a rat according to a method shown in Example 5. That is, 5mm length of fragment is cut off from each specimen and ring shape fragment is prepared and sterilized by gas. These specimens are inserted under dorsal subcutaneous tissue of a rat and observed after one week. As the results, retention of exudates in circumference is not observed in any specimen. The obtained results clearly indicate that by providing a medical material with hydrous property by different means, the medical material can contain physiological saline and obtain flexibility without using glycerin. Accordingly, said medical material does not cause a problem of handling property, further, does not cause retention of exudates in circumference when it is implanted in a living body.

### Example 22

In afore mentioned Example 20, atelocollagen is succinized. In this Example, partially sulfonized chitosan is used and show an example that the isoelectric point is adjusted to pH6.0 or more acid side by making a bioabsorbable material possess electric charge group. It is well known that when chitosan is sulfonized, the sulfonized chitosan becomes to behave like as heparin, and there are many method for sulfonization. In this Example, sulfonized chitosan is prepared according to sulfatized polysaccharides manufacturing method disclosed in JPH09-510493 publication. An artificial blood vessel is coated by mixture of ordinary chitosan and sulfatized chitosan. Isoelectric point of the prepared artificial blood vessel is pH3.5. The prepared artificial blood vessel is used as a specimen, and is estimated according to estimation methods mentioned in Example 20 and Example 21, and similar results are obtained. The obtained results clearly indicate that even if the method for providing with electric charge group is changed, hydrous property can be improved, accordingly flexibility can be obtained. Therefore, it can be said that problem of retention of exudates which is a side effect for using large amount of softening agent and/or moisturizing agent can be dissolved.

### Example 23

Example indicates that a medical material can be hydrous state when a bioabsorbable material has electric charge group and isoelectric point is basic side than pH9.0. In afore mentioned Example 20, atelocollagen is used as a coating material, however, in this Example, protamine sulfate powder is dissolved in atelocollagen solution by 1% ratio and mixed together then a specimen is coated with this solution. Then crosslinked by an epoxy compound same as Example 20. Isoelectric point of coating material mainly composed of atelocollagen is pH9.9. The prepared specimen is lypophilized and sterilized by gas, and as shown in Example 20, the specimen is hardened and is in state of easy to be broken. Then, physiological saline is sprinkled on the specimen, and the specimen absorbs physiological saline gradually and become flexible. It takes about 1minute and 40 seconds, however, flexibility after water absorption is almost same as to a specimen which uses glycerin. These results make it clear that by making a bioabsorbable material possesses electric charge group and adjusting isoelectric point to pH9.0 or more basic side, a specimen obtains flexibility by being hydrous state and does not cause problem in handling property, even if glycerin is not used. That is, it becomes clear that even if content of a softening agent and/or a moisturizing agent is less than 20 weight %, flexibility can be obtained by such a different means.

### Example 24

The specimen prepared in afore mentioned Example 23 and estimated in dorsal subcutaneous tissue of a rat according to a method shown in Example 5. That is, 5mm length of fragment is cut off from each specimen and ring shape fragment is prepared and sterilized by EOG gas. These specimens are inserted under dorsal subcutaneous tissue of a rat and observed after one week. As the results, retention of exudates in circumference is not observed in any specimen. The obtained results clearly indicate that by providing a medical material with hydrous property by different means, the medical material can contain physiological saline and obtain flexibility without using a softening agent and/or a moisturizing agent such as glycerin. Accordingly, said medical material does not cause a problem of handling property, further, does not cause retention of exudates in circumference when it is implanted in a living body.

### Comparative Example 7

Comparison results of crosslinking state by an epoxy compound of the present invention with crosslinking state by glutaraldehyde which is typical crosslinking agent of conventional art is shown in this Example. A specimen is prepared using carboxymethyl chitosan by same method mentioned in Example 1 except using glutaraldehyde instead of epoxy compound as a crosslinking agent. Same as a case of epoxy compound, glutaraldehyde is dissolved in ethanol. And, same as a case of epoxy compound, concentration is set to 3% solution, and crosslinking time is set to 5 hours. Prepared specimen is lypophilized and is very strongly hardened, looses flexibility and is in state of easy to be broken. Then the specimen is soaked into distilled water, however, the specimen does not absorb water and just floating on water. Then the specimen is sunk in water by force for 3 hours, however does not display flexibility, When this state is compared with the state that in cases of Example 1-5, epoxy compound is used as a crosslinking agent and flexibility is obtained by being in hydrous state, it is understood that flexibility by being in hydrous state cannot be obtained by crosslinking using glutaraldehyde which is conventional art.

### Example 25

Example showing that water absorbing ability changes along with the change of hydroxility of a bioabsorbable material after crosslinked. An artificial blood vessel coated with atelocollagen is prepared according to a method of Example 1. Hydroxility of the specimen before crosslinking is measured by an Automatic Hydroxility Measureing Apparatus which is a product of Electric Chemical Systems Co., Ltd. Obtained hydroxility is 25KOHmg/g. Then the specimen is crosslinked by epoxy compound according to a method mentioned in Example 1. After crosslinking, hydroxility becomes 67KOHmg/g. In this state, water absorbing ability is checked. The specimen absorbs physiological saline and becomes flexible within 3 minutes. That is, it becomes clear that even if content of a softening agent and/or a moisturizing agent is less than 20 weight %, flexibility can be obtained by such a different means.

### Comparative Example 8

Comparative Example showing that water absorbing ability changes along with the change of hydroxility of a bioabsorbable material after crosslinked. An artificial blood vessel coated with atelocollagen is prepared according to a method of Example 1. Hydroxility of the specimen before crosslinking is 25KOHmg/g. Then the specimen is crosslinked by glutaraldehyde according to the method mentioned in Comparative Example 7. After crosslinking, hydroxility becomes 13KOHmg/g. In this state, water absorbing ability is checked. The specimen absorbs only small amount of physiological saline and does not become flexible.

### Example 26

As an example of a non-bioabsorbable porous substrate, fibrous material, which is easy to produce various porous state, is selected. First of all, a plain weave cloth using polyester fiber of 1.2 dtex, which is ordinary used in prior art, as main structure is prepared. Water permeability of the cloth is about 500ml. Then, 5 pieces of fragment specimen of 2cm width and 15cm length are cut off to wale direction and to course direction from the cloth, and measured by "45degree Cantilever Bending Resistance Measuring Method". This method uses simple apparatus, concretely uses an inspection table having inclination of 45 degree at the end of flat surface. Specimen is pushed out from flat surface to inclined surface. The cloth specimen bends by gravity and the end of the specimen contacts with inclined surface of the table. The moved distance of the cloth specimen is measured, and the distance (mm) indicates bending resistance of the specimen. In the case of hard cloth, the distance is long, while, in a case of flexible cloth, the distance is short. As the method to prepare a fragment specimen, following method is applied. On the plain weave cloth using 1.2 dtex polyester fiber as main structure, non woven cloth made of 0.15 dtex polyester very fine fiber is piled and high pressure water is blown, thus said two materials becomes one body. By this method, very fine fibers can be interlaced maintaining interstices between fibers by not forming closest packing state. And, at this process, by selecting amount of non woven cloth, interlaced amount of very fine fiber can be voluntarily adjusted. When the amount of non woven cloth is large, weight becomes different, therefore, measurement becomes inaccurate. However, in the region where the amount is small, weight of non woven cloth can be ignored regarding it within an error. The water permeability of the prepared cloth is 480ml.

Cantilever value of the structural cloth is measured and result of 15mm by average is obtained.

Then the cloth to which very fine fibers are interlaced is measured. When amount of very fine fiber is increased, there is a point where Cantilever value changes to 12mm. After that, Cantilever value becomes low along with the increase of amount of very fine fibers. Further, at the point where Cantilever value is changed, softness improving effect by fiber slipping effect caused by containing very fine fibers is displayed. The specimen whose Cantilever value is changed to 12mm is embedded in resin, cut off to 3micron thickness by glass knife and a fragrant is prepared. The fragrant is observed by an optical microscope of 200 magnification and the number of very fine fibers which are interlaced to a substrate is measured, and the very fine fibers occupy more than 5% in average. The very fine fibers spread adequately and there is a interstice of more than 3 micron between fibers. That is, the state is not in closest packing state. According to said results, it becomes clear that the flexibility of porous substrate made of fiber can be improved by containing more than 5% of very fine fibers by not state of closest packing.

Water permeability of a specimen containing 5% of very fine fibers is about 480ml, and not so different from that of substrate. That is, it becomes possible that by interlacing small amount of very fine fibers to a substrate, very fine fibers are interlaced together with without forming closest packing state and realize very fine fibers slipping phenomenon and provide flexibility with the cloth specimen.

### Example 27

A tube of 8mm inside diameter is prepared using "non-bioabsorbable porous substrate made of fiber containing more than 5% of very fine fibers" which is prepared in Example 26, and a bioabsorbable material is combined to the tube, thus a hybrid medical material for in vivo implantation is prepared. As a typical nature originated material of a bioabsorbable material, caboxymethylchitosan is used. Because isoelectric point of said caboxymethylchitosan is around pH5.0, it is dissolved into hydrochloric acidity water of pH5.0 by 5% weight ratio. Then, it is injected into endocavitas of the prepared tube by a syringe, continuously pressed by 3 atoms for 2 minutes and rub the tube. Thus, caboxymethylchitosan is impregnated into interstices of fiber in wall of artificial blood vessel, then the artificial blood vessel is dried by air. Polyepoxy compound (Nagase Kasei Co., Ltd., Osaka Japan) EX313 Glycerol Polyglycidyl Ether is dissolved in isopropylalcohol by 3 volume %, then the artificial blood vessel in which caboxymethylchitosan is impregnated is dipped in said solution and crosslinked at room temperature for 24 hours. Then the artificial blood vessel is washed by stream, lyophilized and sterilized in an autoclave. After that, a cross sectional view of the prepared artificial blood vessel is observed by an optical microscope. Concretely, the artificial blood vessel is embedded in Technovit 7100 (Heraeus Kulzer GmbH & Co. KG), which is hydrophilic resin, sliced with a glass knife and a fragment of about 3 micron thickness is prepared. Cross sectional view is observed by an optical microscope by 40-400 magnification. According to the observation result, state that caboxymethylchitosan is impregnated by scattering state in the intersices of very fine fiber and ordinary thickness fiber. Caboxymethylchitosan part of the artificial blood vessel prepared by above mentioned method is analyzed by an Automatic Hydroxility Measureing apparatus which is a product of Electric Chemical Systems Co., Ltd. And obtained hydroxility is 78KOHmg/g.

When the artificial blood vessel is contacted with physiological saline, it becomes flexible immediately, and there is no problem in handling property as an artificial blood vessel. Further, when the artificial blood vessel is rubbed, coming off phenomenon of caboxymethylchitosan is not observed.

### Example 28

An artificial blood vessel substrate is prepared by mix weaving of very fine fibers an ordinary thickness fiber, which is a substrate made of synthetic fiber containing more than 5% of 0.5 dtex thickness very fine fiber. The method for preparation is same as that of Toray Graft (product of Toray Co., Ltd.: water permeability is 100ml), which is a conventional art. Although Toray Graft is not currently used in actual clinic, this is a weaving structure which uses polyester fiber as the main structure, and polyester more than 5% of very fine fibers of 0.15dtex are woven together so as to interlace to the main structure. Therefore, this product is instructive as an example of an artificial blood vessel which is characterized as low porosity and containing very fine fibers. However, in a case of Toray Graft, which is a conventional art, has a tendency to become closest packing condition and has a risk that the substrate becomes hard. Therefore, an artificial blood vessel to be used in this Example is prepared considering not to form closest packing condition by performing elaborately a raising process and a blowing process of high pressure water. Accordingly, it becomes possible to prepare an artificial blood vessel having flexibility as a structure by slipping phenomenon between very fine fibers, even if the cloth is woven very closely. The artificial blood vessel is called as "very fine fiber artificial blood vessel".

Same as to afore mentioned Example, as a typical nature originated material of a bioabsorbable material, caboxymethylchitosan is used. Because isoelectric point of said caboxymethylchitosan is around pH5.0, it is dissolved into hydrochloric acidity water of pH5.0 by 5% weight ratio. Then, it is injected into endocavitas of the very fine fibers artificial blood vessel by a syringe, continuously pressed by 3 atoms for 2 minutes and rub the tube. Thus, caboxymethylchitosan is impregnated into interstices of fiber in wall of artificial blood vessel, then the artificial blood vessel is dried by air. Polyepoxy compound (Nagase Kasei Co., Ltd., Osaka Japan) EX313 Glycerol Polyglycidyl Ether is dissolved in isopropylalcohol by 3 volume %, then the artificial blood vessel in which caboxymethylchitosan is impregnated is dipped in said solution and crosslinked at room temperature for 24 hours. Then the artificial blood vessel is washed by stream, lyophilized and sterilized in an autoclave. After that a cross sectional view of the prepared artificial blood vessel is observed by an optical microscope. Concretely, the artificial blood vessel is embedded in Technovit 7100 (Heraeus Kulzer GmbH & Co. KG), which is hydrophilic resin, sliced with a glass knife and a fragment of about 3 micron thickness is prepared. Cross sectional view is observed by an optical microscope by 40-400 magnification. According to the observation result, state that caboxymethylchitosan is impregnated by scattering state in interstices between very fine fiber and ordinary thickness fiber. Caboxymethylchitosan part of the artificial blood vessel prepared by above mentioned method is analyzed by an Automatic Hydroxility measureing apparatus which is a product of Electric Chemical Systems Co., Ltd. And obtained hydroxility is 72KOHmg/g.

When the artificial blood vessel is contacted with physiological saline, it becomes flexible immediately, and there is no problem in handling property as an artificial blood vessel. Further, when the artificial blood vessel is rubbed, coming off phenomenon of caboxymethylchitosan is not observed.

Secondary, in this Example, although epoxy compound EX313 is used instead of EX-810 which is used in Example 1, similar results are obtained. Therefore, it become clear that even if a kind of epoxy compound is changed, there is no influence to the artificial blood vessel and hydrous property is good, further the artificial blood vessel becomes flexible by being in hydrous state.

### Comparative Example 9

As a comparative example of an artificial blood vessel made of synthetic fiber not containing very fine fibers of 0.5 dtex thickness, an artificial blood vessel (water permeability is 50ml) which is a product of Ube Kosan Co., Ltd., is used. Compared with very fine fiber artificial blood vessel mentioned in Example 24, this artificial blood vessel is hard and handling property is not good. Then according to same method mentioned in Example 27, caboxymethylchitosan is coated. As a crosslinking agent, polyepoxy compound is used. The specimen, not containing glycerin, is lyophilized and sterilized by gas. The specimen becomes very hard, and will be broken easily when transformed. Secondary, when the specimen is soaked into physiological saline, physiological saline penetrates into inside of the specimen and flexibility is recovered. However, although it becomes flexible to the state of before atelocollagen coating, more flexibility cannot be obtained. That is, said flexibility does not reach the level of the flexibility of the artificial blood vessel using very fine fibers shown in Example 26. Considering this result and Example 27, for the purpose not to lose flexibility and to protect retention of exudates in circumference even if the content of glycerin is decreased, it is effective to investigate substrate besides investigate a method for treatment of bioabsorbable material.

### Example 29

In afore mentioned Example1, example as an artificial blood vessel is indicated. In this Example, an example used as a patch material for a part of heart wall is shown.

An artificial blood vessel prepared in Example 2 using carboxymethyl chitosan is cut to vertical direction and a patch of 4cm length and 2.5cm width is prepared. Glycerin is penetrated in this specimen so as the content to be 10% to the total weight and sterilized by gas. The specimen is slightly hard in this stage, however, there is no anxiety to be broken by pinching with tweezers or coming off of coated material. When the specimen is dipped in physiological saline, it sinks into physiological saline immediately and becomes flexible same as the level when 20 weight % of glycerin is contained. And the specimen is not broken by pinching with tweezers or the coating material does not come off.

Secondly, in clean condition, left chest of dog is opened under general anesthesia, pericardium is incised and expose a base of pulmonary artery and incise said part by using a partial blocking clamp, then the prepared patch is implanted as a right ventricle wall patch. At the implantation, handling of the patch is good, and flexibility is not inferior to patch material containing large amount of glycerin which is produced by conventional art. After 2 weeks from the operation, the dog is generally anesthetized again and the implanted part is observed. In circumference of patch material, young bonding tissue is stuck with pericardium, and retention of exudates is not observed.

### Example 30

Example used to a part of pericardium as a membrane state patch same as to aforementioned Example 29 is shown. To said membrane specimen, glycerin of less than 20 weight % to total amount, concretely 10 weight % of glycerin is contained. In clean condition, left chest of dog is opened under general anesthesia, pericardium is incised by 2cm × 4cm width and the prepared patch is implanted at the position. At the implantation, handling of the patch is good, and flexibility is not inferior to patch material containing large amount of glycerin which is produced by conventional art. After 2 weeks from the operation, the dog is generally anesthetized again and the implanted part is observed. In circumference of patch material, young bonding tissue is stuck with pericardium, and retention of exudates is not observed.

### Comparative Example 10

An artificial blood vessel containing large amount of glycerin produced by conventional art is cut opened and a patch shape specimen is prepared. In this Comparative Example, same experiments as mentioned in Example 28 are performed. Hemashield artificial blood vessel which is used in Comparative Example 1 is selected as a comparison. This artificial blood vessel is coated with collagen, and the collagen is crosslinked by formaldehyde. Since glycerin is impregnated in this artificial blood vessel, it is flexible and handling property is excellent, therefore, this product is used in clinic very often. This artificial blood vessel of 10mm inner direction and 4cm length is cut to vertical direction and a patch specimen is prepared. Glycerin content of this specimen is same as shown in Comparative Example 1, that is, glycerin content to total weight (a) is 41.8%. This specimen is excellent at flexibility and handling property is good. Same as the method mentioned in Example 29, this specimen is implanted as a right ventricle wall patch in a dog. Handling property during the operation is good and there is no problem. After 2 weeks from the operation, the dog is generally anesthetized again and the implanted patch part is observed. At the implanted part, thick tissue is risen and when the tissue is broken, exudates oozes out. At outside of the patch accretion of tissue cannot be observed, and there is no finding to conjecture migration of cell. Accordingly, it becomes clear that in a case of specimen of conventional art which contains large amount of glycerin, when it is used as a patch, retention of exudates is caused in the circumference.

### Example 31

In this Example, a method for preparation of a bioabsorbable material characterized that the material becomes flexible by sinking physiological saline and is excellent in handling property without containing large amount of glycerin and does not cause retention of exudates in circumference when implanted in a living body. As mentioned in Example 1, if amount of glycerin contained in a specimen is less than 20 weight % to total weight, the specimen does not cause retention of exudates when it is implanted in a living body. However, in dry state it is hard and handling property is not so good. Especially, in a case when glycerin content is small or does not contain glycerin, the specimen is too hard and is difficult to handle it at actual implanting operation. However, if the specimen is contacted with physiological saline before use, the specimen becomes flexible by water absorption after few moment, and almost same flexibility as obtained by containing more than 20 weight % of glycerin. Especially, in the specimens shown in Examples 14-20, obtaining of flexibility by being hydrous state is effective. Further, same flexibility can be obtained by sprinkling or soaking in physiological saline and handling property becomes good.

### Comparative Example 11

As a typical example of conventional art containing glycerin, Hemashield artificial blood vessel which is used in Comparative Example 1 is used. Before actual use for implanting it in a living body, it is soaked into physiological saline, however, since it is already flexible before actual use, effect by obtaining flexibility by soaking or by being hydrous state cannot be confirmed.

### Example 32

In this Example, following phenomenon will be indicated. That is, even if a medical material for in vivo implantation containing large amount of glycerin, glycerin content can be decreased by washing it with solvent, concretely with physiological saline, and if content of glycerin is adjusted less than 20 weight % to total weight, side effect of glycerin disappears. As an example containing large amount of glycerin and produced by conventional art, Hemashield graft which is mentioned in Comparative Example 1 is used. As mentioned in Comparative Example 1, this specimen has a problem of retention of exudates in circumference after implantation. Therefore, "30 minutes washing method" which dips Hemashield graft in physiological saline just before the implantation is performed. Concretely, Hemashield graft is dipped into 500ml of physiological saline. One time dipping of less than 10 minutes is made. As a result, 97% of contained glycerin is removed and only 3 % is remaining. That is, the specimen is within the region of less than 20 weight %, which is the essential point of the present invention. Regarding implantation experiment into a living body, after 2 weeks, retention of exudates in circumference is not confirmed. Above mentioned results indicate that by using a preparation method of a medical material for in vivo implantation comprising washing the medical material for in vivo implantation with physiological saline just before implantation and washing out glycerin, thus the side effect of glycerin is protected.

### Example 33

In accordance with aforementioned Example 32, this Example will indicate following phenomenon. That is, even if a medical material for in vivo implantation containing large amount of glycerin, glycerin content can be decreased by washing it with solvent, concretely with physiological saline, and if content of glycerin is adjusted less than 20 weight % to total weight, side effect of glycerin disappears. As an example containing large amount of glycerin and produced by conventional art, Interguard graft which is mentioned in Comparative Example 3 is used. As mentioned in Comparative Example 3, this specimen has a problem of retention of exudates in circumference after implantation. Therefore, the method of "washing" which is shown in Example 13 is applied. As a result, 98.6% of contained glycerin is removed and only 1.4 % of glycerin is remaining. According to the result of implantation experiment in a living body, retention of exudates in circumference is not observed. Above mentioned results indicate that by using a preparation method of a medical material for in vivo implantation comprising washing it with physiological saline just before implantation so as to wash out glycerin, side effect of glycerin can be protected.

### Example 34

Experiment to confirm how many washing time is necessary in physiological saline is performed in this Example. Hemashield graft which is used in comparative Example 1 is used. Three pieces of specimen are prepared, and dipped into 500ml of physiological saline and the dipping process is repeated for 10 times. It becomes clear that more than 90% of glycerin is removed only by first dipping. This result is commonly obtained to each thee specimens. However, amount of glycerin remained in the three artificial blood vessel specimens are not same. This phenomenon indicates that the removal of glycerin is influenced by dipping condition, shaking condition or rubbing condition. However, even if the remaining amount is uneven, it become clear that the amount of glycerin can be adjusted to less than 20weight % by one dipping, namely by one washing, and this adjusting method is effective.

### Example 35

As a method for washing to decrease amount of glycerin in clinic, following method is recommendable. That is, considering a case that an artificial blood vessel is long, "30 minutes washing method", characterizing by dipping an artificial blood vessel into about 1000ml of physiological saline, shaking lightly, then wrapping it by dry gauze and removing water by pushing it lightly is recommended. Actual time required for this method is less than 10 minutes. By this method above mentioned Hemashield graft specimen is washed, and content of glycerin is decreased less than 5% by one dipping. According to an animal experiment, it become clear that three times repetition of this action is sufficient.

### Example 36

A specimen of Hemashield artificial blood vessel whose glycerin content is decreased to less than 20 weight % by the method mentioned in afore mentioned Example 35, concretely an artificial blood vessel whose glycerin content is decreased to less than 4 weight % is implanted into a descending aorta of three beagle dogs. After 2 weeks, the implanted artificial blood vessel is observed, and retention of exudates is not observed in circumferences of the artificial blood vessel. Therefore, it is understood that the preparation method mentioned in Example 34 is effective to reduce the problem of retention of exudates which is a side effect of glycerin.

### Example 37

In a case of use in clinic, a method to decrease amount of glycerin in artificial blood vessel which is already shunted is shown. This method is also an example to use afore mentioned "30 minutes washing method". As an example of medical material containing large amount of glycerin, Hemashield artificial blood vessel shown in Comparative Example 1 is used. After picking up the artificial blood vessel from package, it is implanted into the descending aorta of three beagle dog without contacting with physiological saline. The artificial blood vessel displays flexibility and handling property is excellent. As mentioned in Comparative Example 1, the artificial blood vessel contains 41.8% of glycerin to total weight. If the artificial blood vessel is implanted as is, complication such as retention of exudates will be caused in circumference of the artificial blood vessel as indicated in Comparative Example 2. In this Example, to an artificial blood vessel in which shunt of blood vessel is performed without washing, 300 ml of physiological saline is repeatingly sprinkled to outside of the an artificial blood vessel like as jet water flow using a syringe, in a state that blood is flowing in the artificial blood vessel before the vilnus of operation of class is closed. It takes about 10 minutes to perform said action. After said treatment, the vilnus of this operation is closed and the operation is finished. After two weeks, a bonding tissue is stuck in circumference of the artificial blood vessel, but retention of exudates is not observed. Accordingly, in a case of a medical material, which is already implanted, if the method for washing is performed, complication can be protected. Thus, the effect of this invention becomes obvious.

### Example 38

In a case of clinical use, effect of a method to decrease the amount of glycerin from an artificial blood vessel which is already inplanted in a living body is investigated by simulate experiment. In this experiment, amount of glycerin is altered. Concretely, Hemashield artificial blood vessel used in Comparative Example 10 is used. As a simulate experiment to simulate implantation of the artificial blood vessel in animal, a pump to be used in a cardiopulmonary bypass is used, and a closed cycle is prepared. In this closed cycle low molecule weight dextran solution is flown as suspected blood and adjusted to average inner pressure 120mmHg. In this state, Hemashield artificial blood vessel is took out from a package and is connected to a part of the cycle so as not to contact with physiological saline and the suspected blood is flown in the artificial blood vessel. Length of the artificial blood vessel is 5cm and weight is 0.7862g. After suspected blood is flown, the artificial blood vessel is washed by jet water flow using a syringe according to a method indicated in Example 37. 300ml of physiological saline is used for this washing. After washing the artificial blood vessel is picked out and amount of glycerin is measured. The amount of glycerin contained in the artificial blood vessel of 5cm length is 0.2911g. As shown in Comparative Example 1, since 41.8 weight % of glycerin is contained in this artificial blood vessel, about 0.3571 glycerin ought to be contained at initial stage. The amount of glycerin is decreased to 0.2911g, therefore, it indicates that only 0.0660g of glycerin is remaining. That is, glycerin content is decreased to 8.4 weight %. Accordingly, even if in the state that blood is flowing, when a method for preparation of washing is used, amount of glycerin can be reduced to the level of less than 20 weight % where is a safety zone. That is, the advantage of the method for preparation of the present invention is proved.

### Example 39

As the next, Example reciting a preparation method of medical material for in vivo implantation will be shown. That is, the method is to oppress excessive swelling of a bioabsorbable material, to push the bioabsorbable material into porous structure of a non-bioabsorbable porous substrate effectively and to enmesh it to the porous structure. As an example, succinized collagen is selected as a bioabsorbable material. Since succinized collagen possesses many carboxyl groups, it charges minus. Therefore, it easily contains water in molecule and swells excessively. First of all, aqueous solution of 1% succinized collagen is pressure pushed into very fine fiber artificial blood vessel of 8mm inside diameter and 6cm length used in Example 27 by 300mmHg pressure. Only succinized collagen enmeshed to the porous structure is remained and other excessive succinized collagen is removed, then lyophilized. And weight of enmeshed succinized collagen is measured. The weight of very fine fibers artificial blood vessel is 0.4213g, and the weight in the state that succinized collagen is enmeshed is 0.5124g, therefore the weight of enmeshed succinized collagen is 0.0911g. Secondary, same kind of artificial blood vessel is used, and at the process of pressure pushing of succinized collagen, table salt is mixed with aqueous solution of succinized collagen so as the content to be 20%, then pressure pushed into the substrate. The weight after lyophilization is 0.9454g. That is, it becomes clear that, although table salt is contained, 0.5322g of succinized collagen is enmeshed. Accordingly, by containing salts such as table salt at the preparation process, a bioabsorbable material which swells easily is tied up and becomes easy to enter into narrow space of a porous substrate.

### Example 40

Same very fine fibers artificial blood vessel used in Example 39 is applied and investigation of preparing process is attempted. The weight of the artificial blood vessel is 0.4846g. As the typical bioabsorbable material which swells easily gelatin is selected. First of all, 10% aqueous solution of gelatin is prepared by heating and is pressure pushed into artificial blood vessel of 8mm inside diameter and 6cm length by 300mmHg pressure. Excessive gelatin stuck to a circumference of the artificial blood vessel is removed, then lyophilized and amount of enmeshed gelatin is measured. The weight of the artificial blood vessel is increased to 0.5837g, therefore, the amount of enmeshed gelatin is 0.0991g. Secondary, same experiment except adding table salt to the aqueous solution of gelatin so as the concentration of table salt to be 20% is attempted. Then the specimen is dipped into cooled distilled water and table salt is removed. After that, lyophilized and the weight of the specimen is measured. According to the measuring result, weight of the artificial blood vessel after pressure pushing is 0.9881g. Therefore, it is confirmed that 0.4145g of gelatin is enmeshed to the artificial blood vessel. Compared with the case which does not use table salt, this case indicates that large amount of gelatin is enmeshed effectively. Accordingly, by containing salts such as table salt at the preparation process, a bioabsorbable material which swells easily is tied up and becomes easy to enter into narrow space of a porous substrate.

### Example 41

Same very fine fibers artificial blood vessel used in Example 39 is used and investigation of preparing process is attempted. The weight of the artificial blood vessel is 0.4928g. As the typical bioabsorbable material which swells easily 2% aqueous solution of carboxymethyl cellulose used in Example 7 is selected. First of all, said aqueous solution of carboxymethyl cellulose is pressure pushed into artificial blood vessel of 8mm inside diameter and 6cm length by 300mmHg pressure. Only enmeshed carboxymethyl cellulose is remained and excessive carboxymethyl cellulose stuck to a circumference of the artificial blood vessel is removed, then lyophilized and amount of enmeshed carboxymethyl cellulose is measured. The weight of the artificial blood vessel is increased to 0.5851g, therefore, the amount of enmeshed carboxymethyl cellulose is 0.0923g. Secondary, same experiment except adding ethanol to the aqueous solution of carboxymethyl cellulose so as the concentration of ethanol to be 20% is attempted. After that, the weight of the specimen is measured. According to the measuring result, weight of the artificial blood vessel after pressure pushing is 0.9112g. Since ethanol ought to be removed from the artificial blood vessel at the lyophilization process, 0.4184g of carboxymethyl cellulose is enmeshed to the artificial blood vessel. Since ethanol ought to be removed from the artificial blood vessel at the lyophilization process, exactly this amount of carboxymethyl cellulose is enmeshed to the artificial blood vessel, and when compared with compared with a case which does not mix ethanol, it is understood that large amount of carboxymethyl cellulose is enmeshed effectively. This result indicates that in preparation process, by containing alcohol such as ethanol, a bioabsorbable material which swells easily is tied up and becomes easy to enter into narrow space of a porous substrate.

### Example 42

As the next, Example reciting a preparation method of medical material for in vivo implantation will be shown. In a case of a bioabsorbable material possessing electric charge group, the method is to oppress excessive swelling of a bioabsorbable material, to push the bioabsorbable material into porous structure of a non-bioabsorbable porous substrate effectively and to enmesh it to the porous structure.

As an example, succinized collagen is selected as a bioabsorbable material. Since succinized collagen possesses many carboxyl groups, it charges minus. Therefore, it easily contains water in molecule and swells excessively. And isoelectric point of it is pH3.3. First of all, aqueous solution of 1% succinized collagen is pressure pushed into very fine fiber artificial blood vessel of 8mm inside diameter and 6cm length used in Example 27 by 300mmHg pressure. Only succinized collagen enmeshed to the porous structure is remained and other excessive succinized collagen is removed, then lyophilized. And weight of enmeshed succinized collagen is measured. The weight of very fine fibers artificial blood vessel is 0.4213g, and the weight in the state that succinized collagen is enmeshed is 0.5124g, therefore the weight of enmeshed succinized collagen is 0.0911g. Secondary, same kind of artificial blood vessel is used, and at the process of pressure pushing of succinized collagen, pH of succinized collagen solution is adjusted to pH3.3 and pressure pushed by same condition into the substrate. The weight after lyophilization becomes 0.9234g. That is, it becomes clear that 0.5322g of succinized collagen is enmeshed. Accordingly, by adjusting pH of solution closely to the isoelectric point at the preparation process, a bioabsorbable material which swells easily becomes easy to enter into narrow space of a porous substrate.

### INDUSTRIAL APPLICABILITY

As mentioned above, in the present invention, in a hybrid medical material for in vivo implantation comprising a softening agent and/or a moisturizing agent, by restricting the content of softening agent and/or moisturizing agent less than 20 weight %, retention of exudates caused in circumference of the medical material can be minimized, accordingly bad influence affected by retention of exudates can be protected, further, flexibility of said medical material for in vivo implantation can be maintained by washing with electrolyte solution such as physiological saline, isotonic solution or distilled water or by impregnating it in said solvents.

## Claims

1. A medical material for in vivo implantation which comprises a hybrid medical material for in vivo implantation comprising a bioabsorbable material which contains a softening agent and/or a moisturizing agent and a non-bioabsorbable porous substrate, wherein content of said softening agent and/or moisturizing agent is less than 20 weight % to the total weight of said hybrid medical material for in vivo implantation.

2. The medical material for in vivo implantation of claim 1, wherein the content of said softening agent and/or moisturizing agent is less than 20 weight % to the total weight of the hybrid medical material for in vivo implantation in actual clinical use.

3. The medical material for in vivo implantation of claim 1 or claim 2, wherein the content of said softening agent and/or moisturizing agent is adjusted to less than 20 weight % to the total weight of the hybrid medical material for in vivo implantation by washing with solvents within 30 minutes.

4. The medical material for in vivo implantation according to anyone of claims 1 to 3, wherein said bioabsorbable material possesses electric charge group.

5. The medical material for in vivo implantation according to anyone of claims 1 to 4, wherein hydroxyl value of said bioabsorbable material is more than 50KOH mg/g.

6. The medical material for in vivo implantation according to anyone of claims 1 to 5, wherein said bioabsorbable material is crosslinked by polyepoxy compound.

7. The medical material for in vivo implantation according to anyone of claims 1 to 6, wherein said non-bioabsorbable porous substrate is made of fiber having very fine fibers of less than 0.5 dtex more than 5% to total numbers of fibers so as not to be in closest packing state.

8. The medical material for in vivo implantation according to anyone of claims 1 to 7, wherein said medical material for in vivo implantation is used as a substitute or a part of a tubular viscus or a tubular tissue in vivo such as an artificial blood vessel or an artificial trachea, or is used in patch form on organs or tissues in vivo as an artificial pericardium, an artificial heart wall, an artificial abdominal wall, an artificial blood vessel wall, an artificial trachea wall, an artificial pleura, an artificial dura matter or an artificial urinary bladder wall.

9. A method for preparation of a medical material for in vivo implantation comprising a bioabsorbable material which contains a softening agent and/or a moisturizing agent and a non-bioabsorbable porous substrate comprising, adjusting content of said softening agent and/or moisturizing agent to less than 20 weight % to the total weight of said medical material by washing said medical material with a solvent of said softening agent and/or moisturizing agent or by impregnating said medical material in the solvent.

10. A method for preparation of a medical material for in vivo implantation comprising a bioabsorbable material and a non-bioabsorbable porous substrate comprising, having a process to contain salt and/or alcohol to a solution or a suspension of said bioabsorbable material, to impregnate and enmesh said bioabsorbable material to the non-bioabsorbable porous substrate so as to oppress swelling of said bioabsorbable material by water, then to remove said salt and/or alcohol.

11. A method for preparation of a medical material for in vivo implantation comprising a bioabsorbable material possessing electric charge group and a non-bioabsorbable porous substrate comprising, having a process to dissolve said bioabsorbable material at nearby isoelectric point so as to oppress swelling of said bioabsorbable material by water, then to adjust hydrogen ion concentration to physiological neutral region.
